# EUROPEAN PATENT APPLICATION

(11) **EP 4 235 873 A1**
(43) Date of publication of application: **30.08.2023**
(21) Application number: 22767249.0
(22) Date of filing: 10.03.2022
(51) Int. Cl.: H01M 4/60, C07D 241/46

(54) **ACTIVE MATERIAL FOR SECONDARY BATTERIES, ELECTRODE FOR SECONDARY BATTERIES, SECONDARY BATTERY AND FLIGHT VEHICLE**

(30) Priority: 12.03.2021 JP 2021040756
(71) Applicant: SoftBank Corp., Tokyo 105-7529 (JP); National Institute Of Advanced Industrial Science and Technology, Chiyoda-ku Tokyo 100-8921 (JP)
(72) Inventor: SAITO, Takaya, Tokyo 105-7529 (JP); NISHIYAMA, Koji, Tokyo 105-7529 (JP); TAKAYANAGI, Yoshiki, Tokyo 105-7529 (JP); MIYAKAWA, Shuntaro, Tokyo 105-7529 (JP); YAO, Masaru, Ikeda-shi, Osaka 563-8577 (JP); KATO, Minami, Ikeda-shi, Osaka 563-8577 (JP); ANDO, Hisanori, Ikeda-shi, Osaka 563-8577 (JP); TAKEICHI, Nobuhiko, Ikeda-shi, Osaka 563-8577 (JP)
(74) Representative: Michalski Hüttermann & Partner Patentanwälte mbB
(86) International application number: PCT/JP2022/010687
(87) International publication number: WO 2022/191295

(57) **Abstract**

An active material used in a secondary battery includes a heterocyclic chemical compound including one or more pyrazine rings and two or more benzene rings, or a salt or derivative thereof. The heterocyclic chemical compound is preferably a chemical compound in which at least 4 oxygen atoms are bonded to the benzene ring. The active material used in the secondary battery may also include phenazine or a salt or derivative thereof. The phenazines are preferably chemical compounds in which at least 4 oxygen atoms are bonded to a benzene ring included in a phenazine structure.

## Description

### BACKGROUND

### 1. TECHNICAL FIELD

The present invention relates to an active material for a secondary battery, an electrode for a secondary battery, a secondary battery, and a flying body.

### 2. RELATED ART

Patent Document 1 discloses an electrode active material for a non-aqueous secondary battery composed of a chemical compound in which a naphthazarin skeleton is condensed with a non-conjugated ring such as a dithiin ring, as an organic material exhibiting oxidation-reduction activity. By using the electrode active material for a non-aqueous secondary battery described in Patent Document 1, a storage cell having an energy density of about 400 [Wh/kg-storage cell] can be manufactured. However, there is a need for further improvement in the energy density of the storage cell.

### PRIOR ART DOCUMENT

### PATENT DOCUMENT

Patent Document 1: Japanese Patent Application Publication No. 2018-085243

### GENERAL DISCLOSURE

A first aspect of the present invention provides an active material for a secondary battery, which is an active material used in a secondary battery. The active material includes a chemical compound represented by, for example, the following general formula (1), general formula (2), general formula (3), general formula (4), general formula (4), general formula (5), general formula (6) or general formula (7), or a salt thereof. 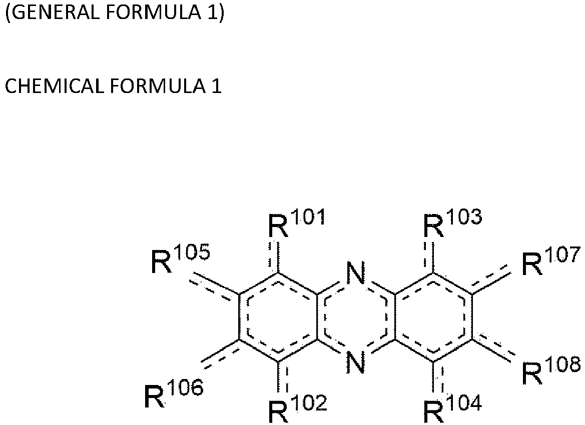 (in the general formula (1), R¹⁰¹ to R¹⁰⁸ each independently represent an oxygen atom, a group represented by -OM, a hydrogen atom or an organic group. M represents a hydrogen atom or a monovalent or bivalent metal atom. Note that, when M is a bivalent metal atom, two adjacent R¹⁰¹ to R¹⁰⁸ are coupled to each other to form a ring represented by - OMO-. A double line represented by a solid line and a dashed line represents a single bond or a double bond. At least four of R¹⁰¹ to R¹⁰⁸ are oxygen atoms, or groups represented by - OM). 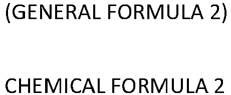 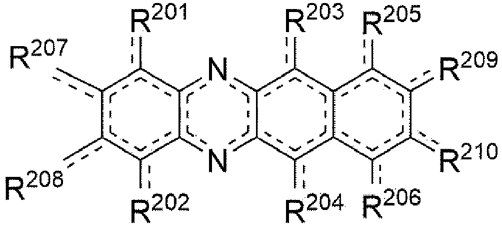 (in the general formula (2), R²⁰¹ to R²¹⁰ each independently represent an oxygen atom, a group represented by -OM, a hydrogen atom or an organic group. M represents a hydrogen atom or a monovalent or bivalent metal atom. Note that, when M is a bivalent metal atom, two adjacent R²⁰¹ to R²¹⁰ are coupled to each other to form a ring represented by - OMO-. A double line represented by a solid line and a dashed line represents a single bond or a double bond. At least four of R²⁰¹ to R²¹⁰ are oxygen atoms, or groups represented by - OM). 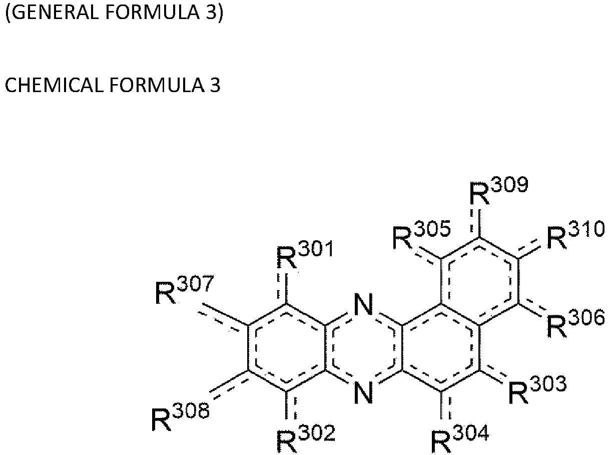 (in the general formula (3), R³⁰¹ to R³¹⁰ each independently represent an oxygen atom, a group represented by -OM, a hydrogen atom or an organic group. M represents a hydrogen atom or a monovalent or bivalent metal atom. Note that, when M is a bivalent metal atom, two adjacent R³⁰¹ to R³¹⁰ are coupled to each other to form a ring represented by - OMO-. A double line represented by a solid line and a dashed line represents a single bond or a double bond. At least four of R³⁰¹ to R³¹⁰ are oxygen atoms, or groups represented by - OM). 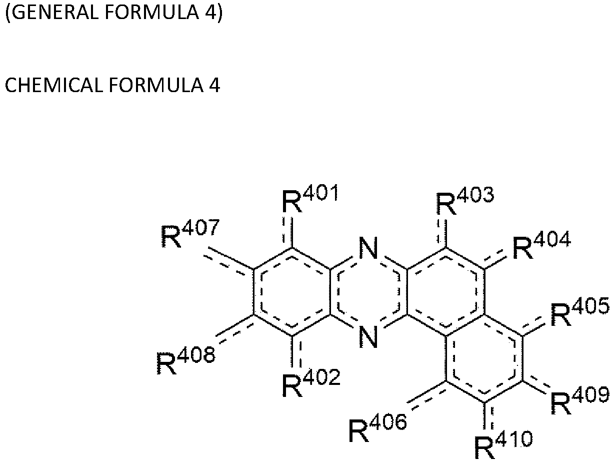 (in the general formula (4), R⁴⁰¹ to R⁴¹⁰ each independently represent an oxygen atom, a group represented by -OM, a hydrogen atom or an organic group. M represents a hydrogen atom or a monovalent or bivalent metal atom. Note that, when M is a bivalent metal atom, two adjacent R⁴⁰¹ to R⁴¹⁰ are coupled to each other to form a ring represented by - OMO-. A double line represented by a solid line and a dashed line represents a single bond or a double bond. At least four of R⁴⁰¹ to R⁴¹⁰ are oxygen atoms, or groups represented by - OM). 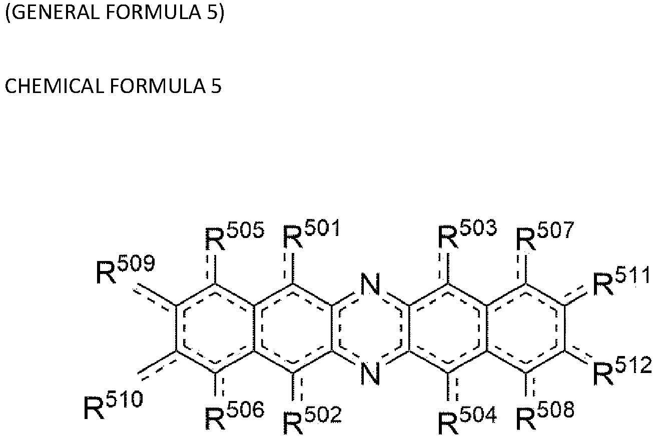 (in the general formula (5), R⁵⁰¹ to R⁵¹² each independently represent an oxygen atom, a group represented by -OM, a hydrogen atom or an organic group. M represents a hydrogen atom or a monovalent or bivalent metal atom. Note that, when M is a bivalent metal atom, two adjacent R⁵⁰¹ to R⁵¹² are coupled to each other to form a ring represented by - OMO-. A double line represented by a solid line and a dashed line represents a single bond or a double bond. At least four of R⁵⁰¹ to R⁵¹² are oxygen atoms, or groups represented by - OM). 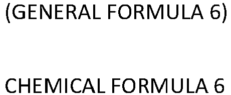 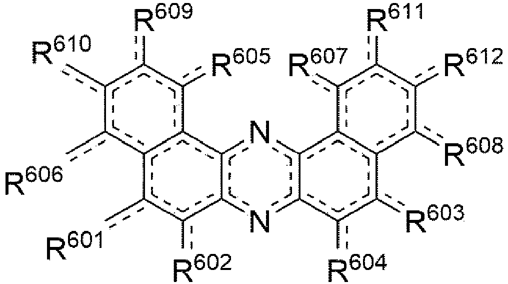 (in the general formula (6), R⁶⁰¹ to R⁶¹² each independently represent an oxygen atom, a group represented by -OM, a hydrogen atom or an organic group. M represents a hydrogen atom or a monovalent or bivalent metal atom. Note that, when M is a bivalent metal atom, two adjacent R⁶⁰¹ to R⁶¹² are coupled to each other to form a ring represented by - OMO-. A double line represented by a solid line and a dashed line represents a single bond or a double bond. At least four of R⁶⁰¹ to R⁶¹² are oxygen atoms, or groups represented by - OM). 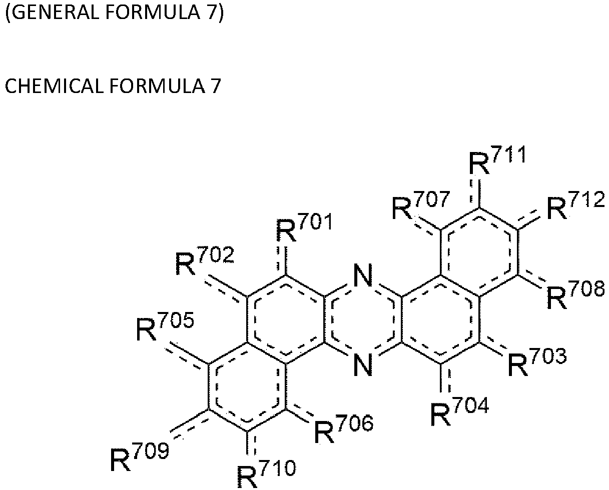 (in the general formula (7), R⁷⁰¹ to R⁷¹² each independently represent an oxygen atom, a group represented by -OM, a hydrogen atom or an organic group. M represents a hydrogen atom or a monovalent or bivalent metal atom. Note that, when M is a bivalent metal atom, two adjacent R⁷⁰¹ to R⁷¹² are coupled to each other to form a ring represented by - OMO-. A double line represented by a solid line and a dashed line represents a single bond or a double bond. At least four of R⁷⁰¹ to R⁷¹² are oxygen atoms, or groups represented by - OM).

In the active material described above, the chemical compound may have symmetry. In the active material described above, the chemical compound may be a chemical compound described below or a fully reduced form or partial reduced form thereof. Note that, in the following chemical formula, R¹ and R² each independently represent a hydrogen atom or an organic group.

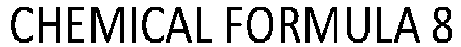

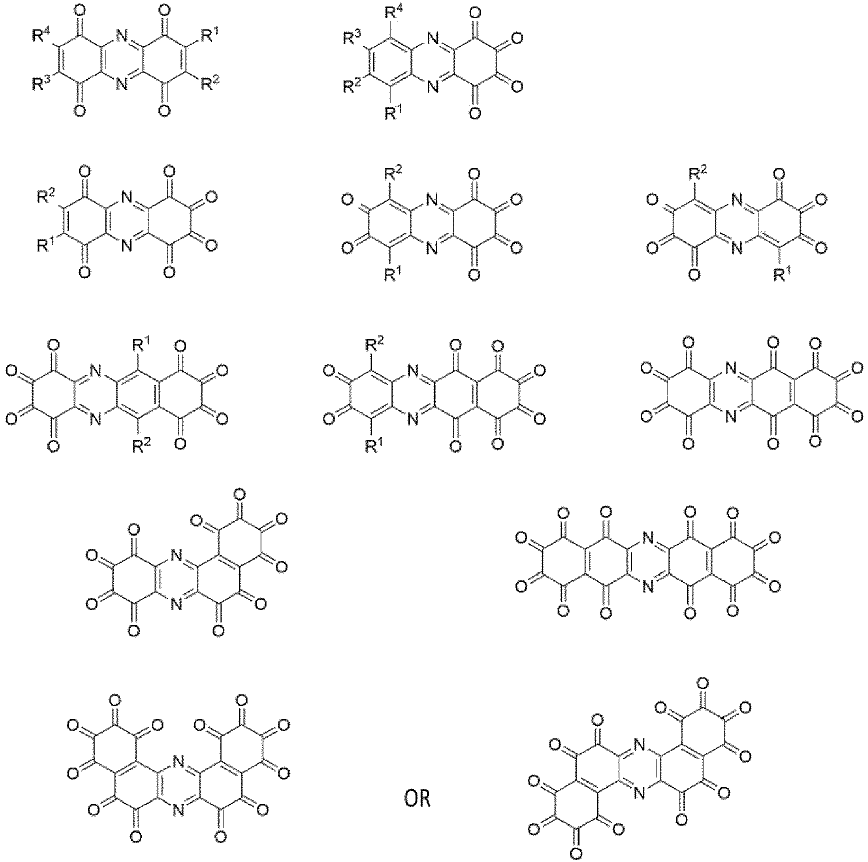

A second aspect of the present invention provides an active material for a secondary battery, which is an active material used in a secondary battery. The active material includes a polymer including a repeating unit represented by, for example, the following general formula (8), general formula (9), general formula (10) or general formula (11), or a salt thereof. 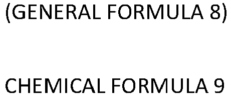 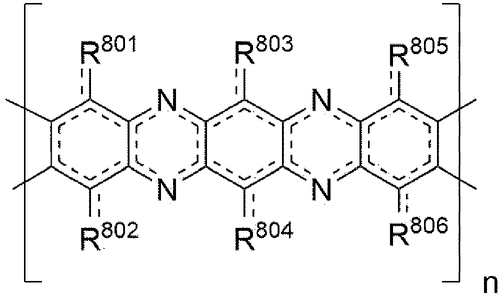 (in the general formula (8), R⁸⁰¹ to R⁸⁰⁶ each independently represent an oxygen atom, a group represented by -OM, a hydrogen atom or an organic group. M represents a hydrogen atom or a monovalent or bivalent metal atom. Note that, when M is a bivalent metal atom, two adjacent R⁸⁰¹ to R⁸⁰⁶ are coupled to each other to form a ring represented by - OMO-. A double line represented by a solid line and a dashed line represents a single bond or a double bond. At least four of R⁸⁰¹ to R⁸⁰⁶ are oxygen atoms, or groups represented by - OM. n is an integer of 2 or larger representing a polymerization degree). 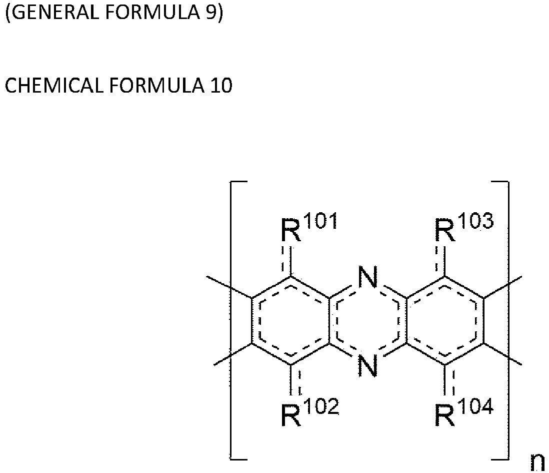 (in the general formula (9), R¹⁰¹ to R¹⁰⁴ each independently an oxygen atom or a group represented by -OM. M represents a hydrogen atom or a monovalent or bivalent metal atom. Note that, when M is a bivalent metal atom, two adjacent R¹⁰¹ to R¹⁰⁴ are coupled to each other to form a ring represented by -OMO-. A double line represented by a solid line and a dashed line represents a single bond or a double bond. n is an integer of 2 or larger representing a polymerization degree). 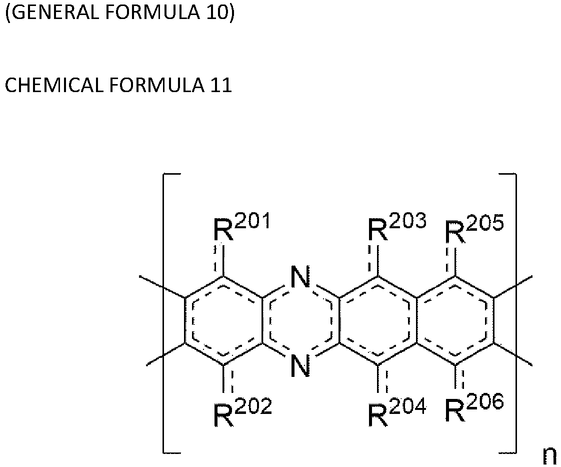 (in the general formula (10), R²⁰¹ to R²⁰⁶ each independently represent an oxygen atom, a group represented by -OM, a hydrogen atom or an organic group. M represents a hydrogen atom or a monovalent or bivalent metal atom. Note that, when M is a bivalent metal atom, two adjacent R²⁰¹ to R²⁰⁶ are coupled to each other to form a ring represented by - OMO-. A double line represented by a solid line and a dashed line represents a single bond or a double bond. At least four of R²⁰¹ to R²⁰⁶ are oxygen atoms, or groups represented by - OM. n is an integer of 2 or larger representing a polymerization degree). 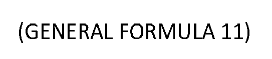 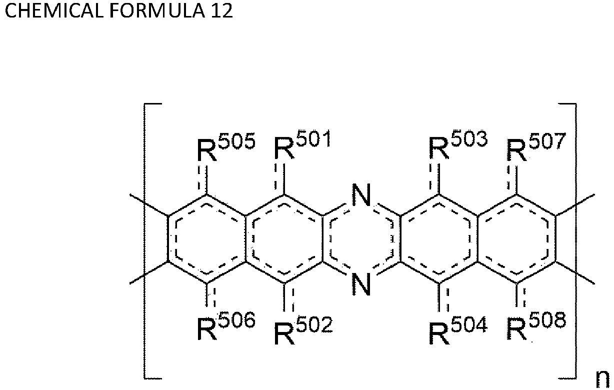 (in the general formula (11), R⁵⁰¹ to R⁵⁰⁸ each independently represent an oxygen atom, a group represented by -OM, a hydrogen atom or an organic group. M represents a hydrogen atom or a monovalent or bivalent metal atom. Note that, when M is a bivalent metal atom, two adjacent R⁵⁰¹ to R⁵⁰⁸ are coupled to each other to form a ring represented by - OMO-. A double line represented by a solid line and a dashed line represents a single bond or a double bond. At least four of R⁵⁰¹ to R⁵⁰⁸ are oxygen atoms, or groups represented by - OM. n is an integer of 2 or larger representing a polymerization degree).

The active material may also include a polymer including a repeating unit represented by the following chemical formula (8-1), or a salt thereof. 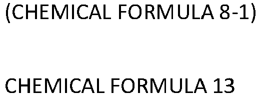 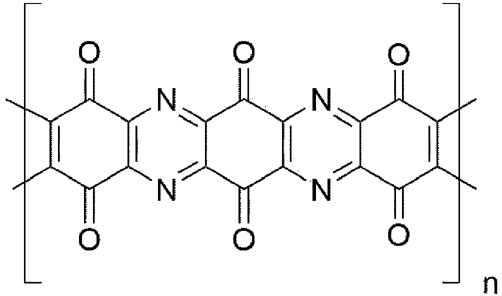 (in the chemical formula (8-1), n is an integer of 2 or larger representing a polymerization degree).

In the active material for a secondary battery according to the first aspect and the second aspect, M may be H, Li, Na, K, Mg, or Ca.

A third aspect of the present invention provides an electrode for a secondary battery. The electrode for a secondary battery includes, for example, the active material for a secondary battery according to the first aspect and the second aspect.

A fourth aspect of the present invention provides a secondary battery. The secondary battery includes, for example, a positive electrode active material layer including a positive electrode active material, a negative electrode active material layer including a negative electrode active material, and an electrolyte. In the secondary battery, the positive electrode active material or the negative electrode active material includes, for example, the active material for a secondary battery according to the first aspect and the second aspect.

In the secondary battery, the positive electrode active material may also include the active material for a secondary battery according to the first aspect and the second aspect. The secondary battery may be a non-aqueous secondary battery.

A fourth aspect of the present invention provides a flying body. The flying body includes, for example, the secondary battery according to the first aspect and the second aspect. The flying body includes, for example, a propulsive force generating apparatus configured to generate propulsive force by using electric energy accumulated in a secondary battery.

The summary of the invention described above does not necessarily describe all necessary features of the embodiments of the present invention. In addition, the present invention may also be a sub-combination of the features described above.

### BRIEF DESCRIPTION OF THE DRAWINGS

Fig. 1 schematically represents an example of a system configuration of a flying body 100.
Fig. 2 schematically represents an example of a storage cell 112.
Fig. 3 is a graph representing a result of Test Example 1 (discharging test of a half-cell using a chemical compound of Example 1 as a positive electrode active material: initial discharging curve).
Fig. 4 is a graph representing a result of Test Example 2 (discharging test of a half-cell using a chemical compound of Example 1 as a positive electrode active material: initial discharging curve).
Fig. 5 is a graph representing a result of Test Example 3 (discharging test of a half-cell using a chemical compound of Example 1 as a positive electrode active material: initial discharging curve).

### DESCRIPTION OF EXEMPLARY EMBODIMENTS

According to the present embodiment, as an active material for a secondary battery, a heterocyclic chemical compound including one or more pyrazine rings and two or more benzene rings, or a salt or derivative thereof is used. As a derivative of a specific organic chemical compound, when the organic chemical compound is taken as a starting material, a chemical compound modified in such a degree without large structural and characteristic modifications in the starting material, such as introduction of a functional group, oxidation, reduction, or replacement of an atom is exemplified. The heterocyclic chemical compound is preferably a chemical compound in which at least 4 oxygen atoms are bonded to the benzene ring. As the active material for a secondary battery, phenazine or a salt or derivative thereof (which may be referred to as phenazines) may be used. The phenazines are preferably chemical compounds in which at least 4 oxygen atoms are bonded to a benzene ring included in a phenazine structure.

In addition, according to the present embodiment, an electrode for a secondary battery including the active material, a secondary battery including the electrode for a secondary battery, and a flying body including the secondary battery are provided. As described above, in the present embodiment, the active material includes one or more pyrazine rings and two or more benzene rings in at least a part of its structure. In addition, at least 4 oxygen atoms are bonded to the two or more benzene rings. This improves the capacity per unit mass of the active material [mAh/g-active material]. As a result, for example, a storage cell having an energy density per unit mass of 500 [Wh/kg-storage cell] or higher is obtained. A secondary battery including the storage cell according to the present embodiment has a high energy density per unit mass, and is thus particularly suitable for use in a flying body.

Hereinafter, the invention will be described through embodiments of the invention, but the following embodiments do not limit the invention according to claims. In addition, not all combinations of features described in the embodiments are essential to the solution of the invention.

In the present specification, when a numerical range is expressed as "A to B", the expression means A or more and B or less. In addition, "substituted or unsubstituted" means "substituted with an optional substituent or not substituted with a substituent". Types of the substituents are not particularly limited unless otherwise mentioned in the specification. In addition, the number of the substituents is not particularly limited unless otherwise mentioned in the specification.

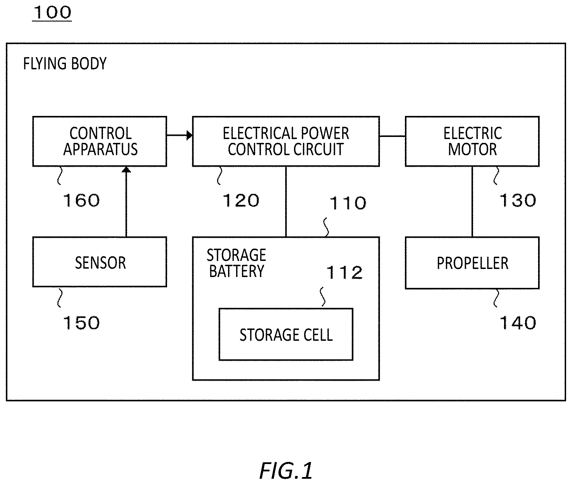

Fig. 1 schematically represents an example of a system configuration of a flying body 100. In the present embodiment, the flying body 100 includes a storage battery 110, an electrical power control circuit 120, one or more electric motors 130, one or more propellers 140, one or more sensors 150, and a control apparatus 160. In the present embodiment, the storage battery 110 has one or more storage cells 112.

In the present embodiment, the flying body 100 flies using the electric energy accumulated in the storage battery 110. Examples of the flying body 100 include an airplane, an airship or a balloon, an aerostat, a helicopter, a drone, and the like.

In the present embodiment, the storage battery 110 receives electric energy from an external charging apparatus (not shown) via the electrical power control circuit 120, and accumulates the electric energy in the one or more storage cells 112. In addition, the storage battery 110 supplies the electric energy accumulated in the one or more storage cells 112 to the electric motor 130 via the electrical power control circuit 120.

In the present embodiment, the storage cell 112 accumulates the electric energy (which may also be referred to as charging of the storage cell 112). In addition, the storage cell 112 discharges the accumulated electric energy (which may also be referred to as discharging of the storage cell 112). The storage cell 112 may be a secondary battery. The storage cell 112 may be a non-aqueous secondary battery.

Examples of the non-aqueous secondary battery include a sodium ion secondary battery, a lithium ion secondary battery, a lithium metal secondary battery, a lithium air secondary battery, a lithium sulfur secondary battery, a magnesium ion secondary battery, an aluminum ion secondary battery, and the like. In addition, the lithium ion secondary battery, which is one aspect of the non-aqueous secondary battery, may have a concept including a non-aqueous lithium ion secondary battery using a non-aqueous electrolyte solution and an all-solid-state lithium ion secondary battery using a solid electrolyte.

For example, as an active material for a secondary battery mounted on a vehicle, a material having a large amount of electric charges that can be accumulated per unit volume is often selected. On the other hand, in the present embodiment, the storage cell 112 is mounted on the flying body 100. For this reason, the active material used in the storage cell 112 is preferably a material having a large amount of electric charges that can be accumulated per unit mass.

A mass energy density of the storage cell 112 is preferably 500 [Wh/kg-storage cell] or higher, more preferably 550 Wh/kg-storage cell] or higher, still more preferably 600 Wh/kg-storage cell] or higher, still more preferably 650 Wh/kg-storage cell] or higher, and still more preferably 700 [Wh/g-storage cell] or higher. Thereby, a storage cell particularly suitable for use as a power source of a flying body is obtained.

A volume energy density of the storage cell 112 may be 300 [Wh/m³-storage cell] or higher and 1200 [Wh/m³-storage cell] or lower, or may be 400 [Wh/m³-storage cell] or higher and 1000 [Wh/m³-storage cell] or lower. When the storage cell 112 is mounted on the flying body 100 as a part of a power source of the flying body 100, the volume energy density of the storage cell 112 may be 600 [Wh/m³-storage cell] or lower or may be 800 [Wh/m³-storage cell] or lower.

The storage cell 112 may have a mass energy density within the numerical range described above and a volume energy density within the numerical range described above. Thereby, a storage cell, which is relatively difficult to use for a power source of a vehicle, can be used as a power source of a flying body. The storage cell 112 will be described in detail later.

In the present embodiment, the electrical power control circuit 120 controls input and output of electrical power of the storage battery 110. The electrical power control circuit 120 may control input and output of the electrical power of the storage battery 110 based on a command from the control apparatus 160. The electrical power control circuit 120 includes, for example, a plurality of switching elements that operate based on a control signal from the control apparatus 160.

In the present embodiment, the electric motor 130 receives the electric energy from the storage battery 110 via the electrical power control circuit 120. The electric motor 130 rotates the propeller 140 by using the electric energy received from the storage battery 110. Thereby, the electric motor 130 can generate propulsive force of the flying body 100 by using the electric energy accumulated in the storage cell 112.

In the present embodiment, the sensor 150 measures various physical quantities relating to a position and a posture of the flying body 100. Examples of the sensor for measuring various physical quantities relating to the position and posture of the flying body 100 include a GPS signal receiver, an acceleration sensor, an angular acceleration sensor, a gyro sensor, and the like. The sensor 150 may measure various physical quantities relating to a state of the storage battery 110. Examples of the sensor for measuring various physical quantities relating to the state of the storage battery 110 include a temperature sensor, a current sensor, a voltage sensor, and the like.

In the present embodiment, the control apparatus 160 controls the flying body 100. The control apparatus 160 may control the input and output of the electrical power of the storage battery 110 by controlling the electrical power control circuit 120. For example, the control apparatus 160 controls an output current, an output voltage, an input current, an input voltage and the like of the storage battery 110. Thereby, the control apparatus 160 can control the position and posture of the flying body 100. The control apparatus 160 may control the position and posture of the flying body 100 by controlling the electrical power control circuit 120 based on the output from the sensor 150.

The storage battery 110 may be an example of the secondary battery. The storage cell 112 may be an example of the secondary battery. The electric motor 130 may be an example of the propulsive force generating apparatus.

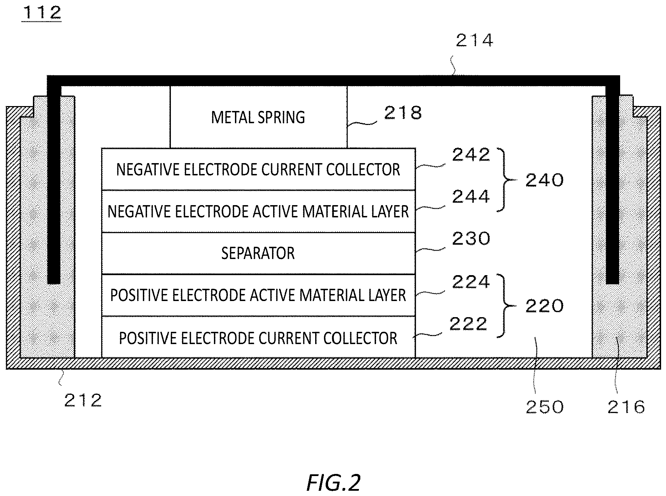

Fig. 2 schematically represents an example of the storage cell 112. In the present embodiment, the details of the storage cell 112 will be described in the case where the storage cell 112 is a coin-type non-aqueous secondary battery, as an example.

### (STORAGE CELL)

In the present embodiment, the storage cell 112 includes a positive electrode case 212, a negative electrode case 214, a sealant 216, and a metal spring 218. In addition, the storage cell 112 includes a positive electrode 220, a separator 230, a negative electrode 240, and an electrolyte solution 250. In the present embodiment, the positive electrode 220 includes a positive electrode current collector 222 and a positive electrode active material layer 224. In the present embodiment, the negative electrode 240 includes a negative electrode current collector 242 and a negative electrode active material layer 244.

In the present embodiment, by assembling the positive electrode case 212 and the negative electrode case 214, a space is formed inside the positive electrode case 212 and the negative electrode case 214. In the space formed by the positive electrode case 212 and the negative electrode case 214, the metal spring 218, the positive electrode 220, the separator 230, the negative electrode 240, and the electrolyte solution 250 are accommodated. The positive electrode 220, the separator 230, and the negative electrode 240 are fixed inside the positive electrode case 212 and the negative electrode case 214 by repulsive force of the metal spring 218.

The positive electrode case 212 and the negative electrode case 214 are each made of a conductive material having, for example, a disk-like thin plate shape. In the present embodiment, the sealant 216 seals a gap formed between the positive electrode case 212 and the negative electrode case 214. The sealant 216 includes an insulating material. The sealant 216 insulates the positive electrode case 212 and the negative electrode case 214.

### (POSITIVE ELECTRODE)

In the present embodiment, the positive electrode current collector 222 holds the positive electrode active material layer 224. Examples of the material of the positive electrode current collector 222 include aluminum, stainless steel, nickel, titanium, alloys thereof, and the like. Examples of the shape of the positive electrode current collector 222 include a foil shape, a mesh shape, a punched-metal shape, an expanded-metal shape, and the like. A thickness of the positive electrode current collector 222 is not particularly limited, but is preferably 5 to 200 µm. The thickness of the positive electrode current collector 222 may also be 6 to 20 µm.

In the present embodiment, the positive electrode active material layer 224 is formed on at least one surface of the positive electrode current collector 222. A thickness of the positive electrode active material layer 224 may be 1 to 300 µm or 2 to 200 µm per surface of the positive electrode current collector 222. The positive electrode active material layer 224 includes, for example, a positive electrode active material and a binding agent (which may be referred to as binder). The positive electrode active material layer 224 may also include a conductive additive.

In one embodiment, the positive electrode active material layer 224 is formed by applying a paste including a material constituting the positive electrode active material layer 224 and an organic solvent onto at least one surface of the positive electrode current collector 222 and drying the paste. The type of the organic solvent is not particularly limited, but examples of the organic solvent include N-methyl pyrrolidone (NMP) and the like. In another embodiment, the positive electrode active material layer 224 is formed by mixing materials constituting the positive electrode active material layer 224 and molding them into a sheet shape, and pressing the sheet-like mixture onto at least one surface of the positive electrode current collector 222.

The positive electrode active material layer 224 may include, as the positive electrode active material, a heterocyclic chemical compound including one or more pyrazine rings and two or more benzene rings, or a salt or derivative thereof. The heterocyclic chemical compound may be a chemical compound including a phenazine structure. The phenazine structure has a structure in which (i) one pyrazine ring and two benzene rings are included and (ii) each of the two benzene rings is coupled to the pyrazine ring by sharing one carbon-carbon bond of the pyrazine ring with the pyrazine ring. The pyrazine ring may be or may not be substituted with an optional functional group. The benzene ring may be or may not be substituted with an optional functional group.

The heterocyclic chemical compound is preferably a chemical compound in which at least 4 oxygen atoms are bonded to the benzene ring. Thereby, the heterocyclic chemical compound can accumulate more electric charges even with a small molecular weight. As a result, a storage cell having a high energy density per unit mass of the storage cell is obtained by using the heterocyclic chemical compound as a positive electrode active material.

The heterocyclic chemical compound is preferably a chemical compound in which oxygen atoms of an even number of 4 or larger are bonded to the benzene ring. The heterocyclic chemical compound may be a chemical compound in which oxygen atoms of an even number of 4 or larger and 12 or smaller are bonded to the benzene ring. This improves the chemical stability of the heterocyclic chemical compound.

The positive electrode active material layer 224 may also include phenazines as a positive electrode active material. The phenazines are preferably chemical compounds in which at least 4 oxygen atoms are bonded to a benzene ring included in a phenazine structure. As described above, this results in a storage cell having a high energy density per unit mass of the storage cell.

The phenazines are preferably chemical compounds in which oxygen atoms of an even number of 4 or larger are bonded to the benzene ring. The phenazines may be chemical compounds in which oxygen atoms of an even number of 4 or larger and 8 or smaller are bonded to the benzene ring. This improves the chemical stability of the heterocyclic chemical compound.

The positive electrode active material described above is composed of, for example, a heterocyclic chemical compound including one or more pyrazine rings and two or more benzene rings, or a salt or derivative thereof. The positive electrode active material mainly includes, for example, a heterocyclic chemical compound including one or more pyrazine rings and two or more benzene rings, or a salt or derivative thereof.

The heterocyclic chemical compound or a salt or derivative thereof and the phenazines are included in any of a reaction starting material, a product and an intermediate product in, for example, at least a discharging reaction of a battery electrode reaction. The details of the heterocyclic chemical compound or a salt or derivative thereof, and the phenazines, which can be used as the positive electrode active material, will be described later.

The positive electrode active material is not limited to the heterocyclic chemical compound and the like. When the storage cell 112 is a lithium ion secondary battery or a lithium metal secondary battery, other examples of the positive electrode active material may include layered oxides such as LiMnO₂, LiNiO₂, LiCoO₂, Li(MnₓNi₁₋ₓ)O₂, Li(MnₓCo₁₋ₓ)O₂, Li(Ni_{y}Co_{1-y})O₂, and Li(MnₓNi_{y}Co_{1-x-y})O₂; solid solutions such as Li₂MnO₃-LiNiO₂, Li₂MnO₃-LiCoO₂, and Li₂MnO₃-Li(Ni_{y}Co_{1-y})O₂; silicates such as Li₂MnSiO₄, Li₂NiSiO₄, Li₂CoSiO₄, Li₂(MnₓNi₁₋ₓ)SiO₄, Li₂(MnₓCo₁₋ₓ)SiO₄, Li₂(Ni_{y}Co_{1-y})SiO₄, and Li₂(MnₓNi_{y}Co_{1-x-y})SiO₄; borates such as LiMnBO₃, LiNiBO₃, LiCoBO₃, Li(MnₓNi₁₋ₓ)BO₃, Li(MnₓCo₁₋ₓ)BO₃, Li(Ni_{y}Co_{1-y})BO₃, and Li(MnₓNi_{y}Co_{1-x-y})BO₃; V₂O₅; LiV₃O₆; MnO, and the like. In the formula, 0<x<1, 0<y<1, 0<x+y<1. These positive electrode active materials may be used alone or in combination of two or more types of positive electrode active materials.

When the storage cell 112 is a sodium ion secondary battery, other examples of the positive electrode active material may include NaFeO₂, NaNiO₂, NaCoO₂, NaMnO₂, NaVO₂, Na(Ni_{X}Mn_{1-X})O₂, Na(Fe_{X}Mn_{1-X})O₂, NaVPO₄F, Na₂FePO₄F, Na₃V₂(PO₄)₃, and the like. In the formula, 0<x<1. These positive electrode active materials may be used alone or in combination of two or more types of positive electrode active materials.

In the present embodiment, the binder binds the materials constituting the positive electrode active material layer 224 (for example, positive electrode active material, conductive additive, and the like) and holds an electrode shape of the positive electrode 220. The type of the binder is not particularly limited, but examples of the binder include polyvinylidene difluoride (PVDF), polytetrafluoroethylene, polyacrylic acid, styrene butadiene rubber, and the like.

In the present embodiment, the conductive additive reduces resistance of the positive electrode 220. The type of the conductive additive is not particularly limited as long as it has desired electronic conductivity, but examples of the conductive additive include a carbon material. Examples of the carbon material include graphite, carbon black (for example, acetylene black, Ketjen black, and the like), cokes, amorphous carbon, carbon fiber, carbon nanotube, graphene, and the like. These conductive additives may be used alone or in combination of two or more types of conductive additives.

When the positive electrode active material layer 224 does not include a conductive additive, a content of the positive electrode active material in the positive electrode active material layer 224 is preferably 40 to 99 mass%, more preferably 70 to 97 mass%, and still more preferably 80 to 95 mass%. A content of the binder in the positive electrode active material layer 224 is preferably 1 to 60 mass%, more preferably 3 to 30 mass%, and still more preferably 5 to 20 mass%.

When the positive electrode active material layer 224 includes a conductive additive, a content of the conductive additive in the positive electrode active material layer 224 is preferably 0.2 to 20 mass%, more preferably 1 to 10 mass%, and still more preferably 2 to 6 mass%. In this case, the content of the positive electrode active material in the positive electrode active material layer 224 is preferably 60 to 98 mass%, more preferably 70 to 96 mass%, and still more preferably 80 to 94 mass%. Further, the content of the binder in the positive electrode active material layer 224 is preferably 1.8 to 39.8 mass%, more preferably 3 to 29 mass%, and still more preferably 4 to 18 mass%.

Note that the content of the binder in the positive electrode active material layer 224 may be 1 to 20 mass%, 2 to 10 mass%, or 3 to 6 mass%. In this case, the remainder of the positive electrode active material layer 224 may be the positive electrode active material or a mixture of the positive electrode active material and the conductive additive.

### (SEPARATOR)

In the present embodiment, the separator 230 separates the positive electrode 220 and the negative electrode 240. The separator 230 secures ionic conductivity between the positive electrode 220 and the negative electrode 240 by, for example, retaining the electrolyte solution. Examples of a material of the separator 230 include polyethylene, polypropylene, ethylene-propylene copolymer, glass, composites thereof, and the like. Examples of a shape of the separator 230 include a microporous film shape, a nonwoven fabric shape, a filter shape, and the like. A thickness of the separator 230 is not particularly limited, but is preferably 10 to 50 µm. An aperture ratio of the separator 230 is not particularly limited, but is preferably 30 to 70%.

### (NEGATIVE ELECTRODE)

In the present embodiment, the negative electrode current collector 242 holds the negative electrode active material layer 244. Examples of a material of the negative electrode current collector 242 include copper, aluminum, stainless steel, nickel, titanium, alloys thereof, and the like. The negative electrode current collector 242 may include a resin support layer and a metal layer arranged on a surface of the support layer. Examples of the resin include polyethylene, polypropylene, polyethylene terephthalate, polyimide, and the like. The metal layer may be a layer made of copper, aluminum, stainless steel, nickel, titanium or an alloy thereof. The metal layer may include a layer made of copper, aluminum, stainless steel, nickel, titanium, or an alloy thereof. The metal layer may be a foil or a plated layer.

When lithium metal is used for the negative electrode active material, the lithium metal can serve as a current collector. For this reason, when the storage cell 112 is a lithium metal secondary battery, the storage cell 112 may not include the negative electrode current collector 242.

Examples of a shape of the negative electrode current collector 242 include a foil shape, a mesh shape, a punched-metal shape, an expanded-metal shape, and the like. A thickness of the negative electrode current collector 242 is not particularly limited, but may be 5 to 200 µm. The thickness of the negative electrode current collector 242 is preferably 6 to 20 µm.

In the present embodiment, the negative electrode active material layer 244 is formed on at least one surface of the negative electrode current collector 242. A thickness of the negative electrode active material layer 244 may be 1 to 300 µm or 2 to 200 µm per surface of the negative electrode current collector 242. The negative electrode active material layer 244 includes, for example, a negative electrode active material and a binder. The negative electrode active material layer 244 may also include a conductive additive.

In one embodiment, the negative electrode active material layer 244 is formed by applying a paste including a material constituting the negative electrode active material layer 244 and an organic solvent onto at least one surface of the negative electrode current collector 242, and drying the paste. The type of the organic solvent is not particularly limited, but examples of the organic solvent include N-methyl pyrrolidone (NMP) and the like. In another embodiment, the negative electrode active material layer 244 is formed by mixing materials constituting the negative electrode active material layer 244 and molding them into a sheet shape, and pressing the sheet-like mixture onto at least one surface of the negative electrode current collector 242.

The negative electrode active material layer 244 may include, as the negative electrode active material, a heterocyclic chemical compound including one or more pyrazine rings and two or more benzene rings, or a salt or derivative thereof. The heterocyclic chemical compound may be a chemical compound including a phenazine structure. The phenazine structure has a structure in which (i) one pyrazine ring and two benzene rings are included and (ii) each of the two benzene rings is coupled to the pyrazine ring by sharing one carbon-carbon bond of the pyrazine ring with the pyrazine ring. The pyrazine ring may be or may not be substituted with an optional functional group. The benzene ring may be or may not be substituted with an optional functional group.

Similarly to the heterocyclic chemical compound described in relation to the positive electrode active material layer 224, the heterocyclic chemical compound that can be used for the negative electrode active material layer 244 is preferably a chemical compound in which at least 4 oxygen atoms are bonded to the benzene ring. The heterocyclic chemical compound is preferably a chemical compound in which oxygen atoms of an even number of 4 or larger are bonded to the benzene ring. The heterocyclic chemical compound may be a chemical compound in which oxygen atoms of an even number of 4 or larger and 12 or smaller are bonded to the benzene ring.

The negative electrode active material layer 244 may also include phenazines as the negative electrode active material. The phenazines are preferably chemical compounds in which at least 4 oxygen atoms are bonded to a benzene ring included in a phenazine structure. The phenazines are preferably chemical compounds in which oxygen atoms of an even number of 4 or larger are bonded to the benzene ring. The phenazines may be chemical compounds in which oxygen atoms of an even number of 4 or larger and 8 or smaller are bonded to the benzene ring.

The negative electrode active material is composed of, for example, a heterocyclic chemical compound including one or more pyrazine rings and two or more benzene rings, or a salt or derivative thereof. The negative electrode active material mainly includes, for example, a heterocyclic chemical compound including one or more pyrazine ring and two or more benzene rings, or a salt or derivative thereof.

The heterocyclic chemical compound or a salt or derivative thereof and the phenazines are included in any of a reaction starting material, a product and an intermediate product in, for example, at least a charging reaction of a battery electrode reaction. The details of the heterocyclic chemical compound or a salt or derivative thereof, and the phenazines, which can be used as the negative electrode active material, will be described later.

The negative electrode active material is not limited to the heterocyclic chemical compound or the like described above. When the storage cell 112 is a lithium ion secondary battery, other examples of the negative electrode active material include (i) graphite, (ii) sintering-resistant carbon or graphitization-resistant carbon, (iii) tin, silicon, and alloys including the same, (iv) SiO, and the like. When a material such as (i) graphite, (ii) sintering-resistant carbon or graphitization-resistant carbon, (iii) tin, silicon and alloys including the same, and (iv) SiO is used as the negative electrode active material, the material may be pre-doped with lithium.

The negative electrode active material may be a lithium-containing material such as metal lithium or a lithium alloy. For example, when the storage cell 112 is a lithium metal secondary battery, metal lithium is used as the negative electrode. These negative electrode active materials may be used alone or in combination of two or more types of negative electrode active materials.

The negative electrode active material layer 244 may also include a lithium metal foil. Thereby, lithium is supplied to the storage cell 112. A thickness of the lithium metal foil may be 1 to 300 µm, 2 to 200 µm, or 3 to 100 µm. The thickness and/or mass of the lithium metal foil may be determined according to the content of the positive electrode active material in the positive electrode active material layer 224.

When the storage cell 112 is a sodium ion secondary battery, other examples of the negative electrode active material include (i) graphite, (ii) sintering-resistant carbon or graphitization-resistant carbon, (iii) tin, silicon and alloys including the same, (iv) titanium oxide, and the like. The negative electrode active material may be a sodium-containing material such as metal sodium and sodium alloy. These negative electrode active materials may be used alone or in combination of two or more types of negative electrode active materials.

In the present embodiment, the binder binds the materials constituting the negative electrode active material layer 244 (for example, positive electrode active material, conductive additive, and the like) and holds the electrode shape of the negative electrode 240. The type of the binder is not particularly limited, but examples of the binder include polyvinylidene difluoride (PVDF), polytetrafluoroethylene, polyacrylic acid, styrene butadiene rubber, and the like.

In the present embodiment, the conductive additive reduces resistance of the negative electrode 240. The type of the conductive additive is not particularly limited as long as it has desired electronic conductivity, but examples of the conductive additive include a carbon material. Examples of the carbon material include graphite, carbon black (for example, acetylene black, Ketjen black, and the like), cokes, amorphous carbon, carbon fiber, carbon nanotube, graphene, and the like. These conductive additives may be used alone or in combination of two or more types of conductive additives.

When the negative electrode active material layer 244 does not include a conductive additive, the content of the negative electrode active material in the negative electrode active material layer 244 is preferably 40 to 99 mass%, more preferably 80 to 98.5 mass%, and still more preferably 90 to 98 mass%. The content of the binder in the negative electrode active material layer 244 is preferably 1 to 60 mass%, more preferably 1.5 to 20 mass%, and still more preferably 2 to 10 mass%.

When the negative electrode active material layer 244 includes a conductive additive, the content of the conductive additive in the negative electrode active material layer 244 is preferably 0.1 to 20 mass%, more preferably 1 to 10 mass%, and still more preferably 2 to 5 mass%. In this case, the content of the negative electrode active material in the negative electrode active material layer 244 is preferably 40 to 98 mass%, more preferably 80 to 97 mass%, and still more preferably 90 to 96 mass%. Further, the content of the binder in the negative electrode active material layer 244 is preferably 1.9 to 59.9 mass%, more preferably 2 to 19 mass%, and still more preferably 2 to 8 mass%.

Note that the content of the binder in the negative electrode active material layer 244 may be 0.1 to 5 mass%, 0.2 to 3 mass%, or 0.5 to 1 mass%. In this case, the remainder of the negative electrode active material layer 244 may be the negative electrode active material or a mixture of the negative electrode active material and the conductive additive.

### (ELECTROLYTE)

In the present embodiment, the electrolyte solution 250 realizes ionic conduction between the positive electrode active material and the negative electrode active material through the electrolyte included in the electrolyte solution 250. According to the present embodiment, as the electrolyte solution 250, a non-aqueous electrolyte solution is used. As the non-aqueous electrolyte solution, a known organic electrolyte solution can be used. For example, when the storage cell 112 is a lithium ion secondary battery or a lithium metal secondary battery, a solution obtained by dissolving (ii) a lithium salt such as lithium perchlorate and LiPF₆ in (i) a solvent composed of one or more of ethylene carbonate, dimethyl carbonate, diethyl carbonate and the like is used as the electrolyte solution 250.

The non-aqueous electrolyte solution contains, for example, a metal salt and a non-aqueous solvent. Examples of the metal salt include a sodium salt, a lithium salt, and the like. Examples of the sodium salt include an inorganic sodium salt such as NaPF₆, NaBF₄, NaClO₄ and NaAsF₆, and an organic sodium salt such as NaCF₃SO₃, NaN(CF₃SO₂)₂, NaN(C₂F₅SO₂)₂ and NaC(CF₃SO₂)₃. Examples of the lithium salt include an inorganic lithium salt such as LiPF₆, LiBF₄, LiClO₄, and LiAsF₆, and organic lithium salt such as LiCF₃SO₃, LiN(CF₃SO₂)₂, LiN(C₂F₅SO₂)₂, and LiC(CF₃SO₂)₃. Examples of the non-aqueous solvent include ethylene carbonate (EC), propylene carbonate (PC), dimethyl carbonate (DMC), diethyl carbonate (DEC), ethyl methyl carbonate (EMC), butylene carbonate (BC), fluoroethylene carbonate (FEC), γ-butyrolactone, sulfolane, acetonitrile, 1,2-dimethoxy methane, 1,3-dimethoxy propane, diethyl ether, tetrahydrofuran, 2-methyl tetrahydrofuran, and mixtures thereof.

A concentration of the sodium salt in the non-aqueous electrolyte solution is not particularly limited, but may be within a range of 0.5 to 4.0 mol/L. The concentration of the sodium salt may be within a range of 0.7 mol/L to 2.0 mol/L, or may be within a range of 1.0 mol/L to 1.5 mol/L. A concentration of the lithium salt in the non-aqueous electrolyte solution is not particularly limited, but may be within a range of 0.5 to 4.0 mol/L. The concentration of the lithium salt may be within a range of 0.7 mol/L to 2.0 mol/L, or may be within a range of 1.0 mol/L to 1.5 mol/L.

### (EXAMPLES OF OTHER EMBODIMENTS)

In the present embodiment, the details of the storage cell 112 have been described in the case where the storage cell 112 is a coin-type secondary battery, as an example. However, the type, structure, and the like of the storage cell 112 are not limited to the present embodiment. In another embodiment, the storage cell 112 may be a cylindrical battery including a wound electrode body in which a positive electrode, a separator, and a negative electrode are wound in a spiral shape. In still another embodiment, the storage cell 112 may be a laminate-type battery in which a laminated electrode body in which a positive electrode and a negative electrode are alternately laminated with a separator being interposed therebetween is sealed with a laminate.

In the present embodiment, the details of the storage cell 112 have been described in the case where the negative electrode 240 includes the negative electrode current collector 242 and the negative electrode active material layer 244, as an example. However, the negative electrode of the storage cell 112 is not limited to the present embodiment. In another embodiment, for example, when the storage cell 112 is a lithium metal secondary battery, metal lithium may be used as the negative electrode.

In the present embodiment, the example of the storage cell 112 has been described in the case where the electrolyte solution 250 is used as the electrolyte of the storage cell 112, as an example. However, the electrolyte of the storage cell 112 is not limited to the present embodiment. In another embodiment, as the electrolyte of the storage cell 112, a solid electrolyte or a gel electrolyte may be used. Examples of the solid electrolyte include an inorganic solid electrolyte such as a Li₂S-P₂S₅ system and a Li₂S-GeS₂-P₂S₅ system.

As described above, the details of the flying body 100 and the storage cell 112 have been described using Figs. 1 and 2. Next, the details of the heterocyclic chemical compound or its salt or derivative, and phenazines, which are used as the positive electrode active material or the negative electrode active material, will be described.

### (I. ACTIVE MATERIAL)

As described above, according to the present embodiment, the heterocyclic chemical compound including one or more pyrazine rings and two or more benzene rings, or a salt or derivative thereof is used as the positive electrode active material or a negative electrode active material (which may be simply referred to as an active material) for a secondary battery. The heterocyclic chemical compound may be a chemical compound including a phenazine structure. The heterocyclic chemical compound is preferably a chemical compound in which at least 4 oxygen atoms are bonded to the benzene ring. The heterocyclic chemical compound may be a chemical compound in which oxygen atoms of an even number of 4 or larger are bonded to the benzene ring.

As the active material for a secondary battery, phenazine or a salt or derivative thereof (which may also be referred to as phenazines) may be used. The phenazines are preferably chemical compounds in which at least 4 oxygen atoms are bonded to a benzene ring included in a phenazine structure. The phenazines may be chemical compounds in which oxygen atoms of an even number of 4 or larger are bonded to the benzene ring.

### (FIRST EMBODIMENT)

In one embodiment, the active material includes a chemical compound represented by the following general formula (1), general formula (2), general formula (3), general formula (4), general formula (4), general formula (5), general formula (6) or general formula (7), or a salt thereof. The chemical compound or salt thereof may be included in any of a reaction starting material, a product and an intermediate product in a discharging reaction of a battery electrode reaction. The chemical compound or salt thereof may be included in any of a reaction starting material, a product and an intermediate product in a charging reaction of the battery electrode reaction.

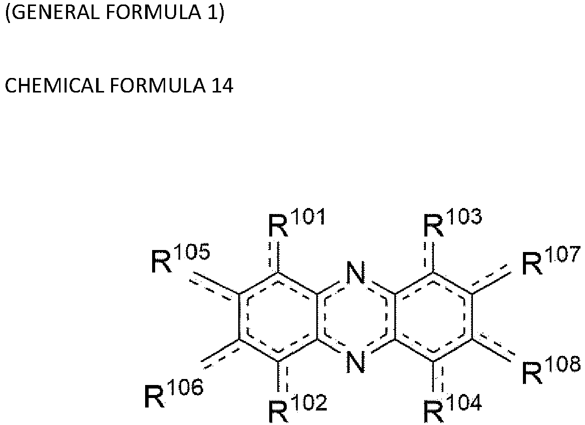

In the general formula (1), R¹⁰¹ to R¹⁰⁸ each independently represent an oxygen atom, a group represented by -OM, a hydrogen atom or an organic group. M represents a hydrogen atom or a monovalent or bivalent metal atom. Note that, when M is a bivalent metal atom, two adjacent R¹⁰¹ to R¹⁰⁸ are coupled to each other to form a ring represented by - OMO-. A double line represented by a solid line and a dashed line represents a single bond or a double bond.

In the present embodiment, at least four of R¹⁰¹ to R¹⁰⁸ are oxygen atoms or groups represented by -OM (which may also be referred to as OM groups). The number of the oxygen atoms or OM groups of R¹⁰¹ to R¹⁰⁸ may be an even number of 4 or larger. R¹⁰¹ to R¹⁰⁸ may be all oxygen atoms or OM groups.

This increases the number of electrons involved in an oxidation-reduction reaction. Specifically, a multi-electron reaction of 4 or more electrons can occur in an oxidation-reduction reaction, and thus the theoretical capacity of the active material increases. For this reason, the active material can accumulate more electric charges even with a small molecular weight. As a result, a storage cell having a high energy density per unit mass of the storage cell is obtained.

In a state in which the active material is fully charged, (i) all of the oxygen atoms or OM groups become oxygen atoms, and (ii) the bond between the oxygen atoms and carbons of the benzene ring included in the phenazine structure becomes a double bond. In this case, the atom represented by M described above is not bonded to a nitrogen atom included in the pyrazine ring of the phenazine structure. For example, no hydrogen ion or metal ion is bonded to the nitrogen atom.

On the other hand, in a state in which the active material is completely discharged, (i) all of the oxygen atoms or OM groups become OM groups, and (ii) the bond between the oxygen atoms of the OM groups and the carbons of the benzene ring included in the phenazine structure becomes a single bond. In this case, the atom represented by M described above is bonded to the nitrogen atom included in the pyrazine ring of the phenazine structure. For example, a hydrogen ion or a metal ion is bonded to the nitrogen atom.

The organic groups represented by R¹⁰¹ to R¹⁰⁸ may be substituted or unsubstituted hydrocarbon groups. The hydrocarbon groups may be monovalent hydrocarbon groups. The organic groups represented by R¹⁰¹ to R¹⁰⁸ may form a ring by two adjacent organic groups being coupled to each other.

Examples of the organic group include a substituted or unsubstituted alkyl group, a substituted or unsubstituted alkenyl group, a substituted or unsubstituted alkynyl group, a substituted or unsubstituted alkoxy group, a substituted or unsubstituted carboxy group, a substituted or unsubstituted alkoxy carbonyl group, a substituted or unsubstituted acyl group, a substituted or unsubstituted acyloxy group, a substituted or unsubstituted aryl group, a substituted or unsubstituted aryloxy group, a substituted or unsubstituted heteroaryl group, a substituted or unsubstituted heteroaryloxy group, a substituted or unsubstituted ester group , a substituted or unsubstituted ether group, a substituted or unsubstituted amino group, a substituted or unsubstituted sulfonic acid group, a substituted or unsubstituted cyano group, a substituted or unsubstituted thioether group, and the like. The organic group may be a monovalent group having an ester bond (-COO-) or a monovalent group having an ether bond (-O-). The organic group may include boron. The organic group may also include boron as a hetero atom.

The organic group may be constituted by one or more atoms selected from the group consisting of carbon, hydrogen, oxygen, nitrogen and boron. These atoms each have a small atomic weight, as compared with, for example, sulfur. For this reason, the organic group is constituted by carbon, hydrogen, oxygen, nitrogen, and boron, so that the molecular weight of the chemical compound used as an active material becomes relatively small. As a result, the mass energy density of the active material or storage cell is improved. The organic group may also be constituted by one or more atoms selected from the group consisting of carbon, hydrogen, oxygen and nitrogen.

In the case where the number of electrons involved in the oxidation-reduction reaction in the active material is the same, when the molecular weight of the active material increases, an amount of electric charges that can be accumulated per unit mass decreases. For this reason, when the organic group is a substituted or unsubstituted hydrocarbon group, the number of carbon atoms in the organic group is preferably 1 to 6, more preferably less than 4, and still more preferably 2 or less. The number of carbon atoms in the organic group may also be one.

Examples of the organic group include a substituted or unsubstituted alkyl group, a substituted or unsubstituted alkoxy group, a substituted or unsubstituted aryl group, a substituted or unsubstituted carboxy group, and the like. In the active material, the smaller the molecular weight and/or size of the organic group is, the lowering in capacity of the storage cell can be suppressed. The alkyl group, alkoxy group, aryl group and carboxy group are relatively small in molecular weight or size. For this reason, when the active material includes these groups, the lowering in capacity of the storage cell including the active material can be suppressed, as compared with a case where the active material includes other organic groups.

Examples of the alkyl group include alkyl groups having 1 to 6 carbon atoms, such as a methyl group, an ethyl group, an n-propyl group, an isopropyl group, an n-butyl group, an isobutyl group, a sec-butyl group, and a tert- butyl group. The number of carbon atoms of the alkyl group is particularly preferably 1 to 3. The alkyl group may be a straight chain alkyl group or a branched chain alkyl group.

Examples of the alkoxy group include alkoxy groups having 1 to 6 carbon atoms such as a methoxy group, an ethoxy group, an n-propoxy group, an isopropoxy group, an n-butyloxy group, an isobutyloxy group, a sec-butyloxy group, and a tert-butyloxy group. The number of carbon atoms of the alkoxy group is particularly preferably 1 to 3. The alkoxy group described above may be a straight chain alkoxy group or a branched chain alkoxy group.

Examples of the aryl group include a phenyl group, a naphthyl group, an anthranyl group, a phenanthryl group, a biphenyl group, and a pyridyl group. The aryl group is particularly preferably a phenyl group.

In the OM group represented by R¹⁰¹ to R¹⁰⁸, M may be H, Li, Na, K, Mg or Ca. The type of metal atom represented by M may be the same as the type of metal atom constituting metal ions moving between the positive electrode and the negative electrode of the secondary battery. For example, when the secondary battery is a lithium ion secondary battery or a lithium metal secondary battery, M represents a hydrogen atom or a lithium atom. When the secondary battery is a sodium ion battery, M represents a hydrogen atom or a sodium atom.

As described above, in the case where the number of electrons involved in the oxidation-reduction reaction in the active material is the same, when the molecular weight of the active material increases, an amount of electric charges that can be accumulated per unit mass decreases. For this reason, the smaller a ratio of the molecular weight of the chemical compound to the number of electrons involved in the oxidation-reduction reaction in the chemical compound represented by the general formula (1) is, the higher the volume energy density of the chemical compound may be. Considering the ease of synthesis and the amount of electric charges that can be accumulated per unit mass, those other than the oxygen atom or OM group of R¹⁰¹ to R¹⁰⁸ are preferably hydrogen atoms.

In the present embodiment, the chemical compound represented by the general formula (1) preferably has symmetry. For example, the chemical compound represented by the general formula (1) has an axis of symmetry, a plane of symmetry, or a center of symmetry.

According to the general formula (1), a fully oxidized form and a fully reduced form of a specific chemical compound are shown. When both the fully oxidized form and the fully reduced form are not radicals, a reversible oxidation-reduction reaction is obtained. When the chemical compound represented by the general formula (1) has symmetry, both the fully oxidized form and the fully reduced form of the chemical compound are not radicals, and thus a reversible oxidation-reduction reaction is obtained. In addition, considering the ease of synthesis, those other than the oxygen atom or OM group of R¹⁰¹ to R¹⁰⁸ are preferably the same.

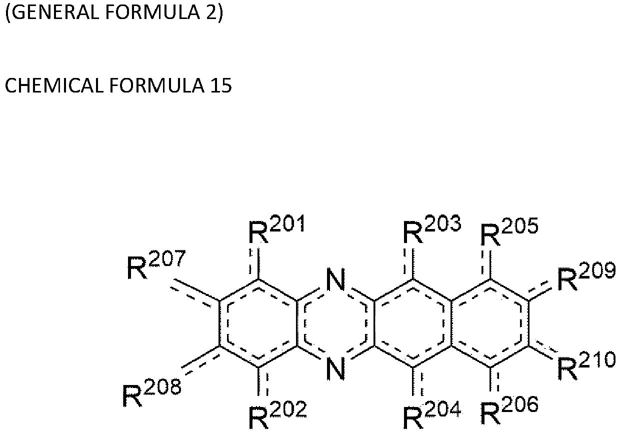

In the general formula (2), R²⁰¹ to R²¹⁰ each independently represent an oxygen atom, a group represented by -OM, a hydrogen atom or an organic group. M represents a hydrogen atom or a monovalent or bivalent metal atom. Note that, when M is a bivalent metal atom, two adjacent R²⁰¹ to R²¹⁰ are coupled to each other to form a ring represented by - OMO-. A double line represented by a solid line and a dashed line represents a single bond or a double bond.

In the present embodiment, at least four of R²⁰¹ to R²¹⁰ are oxygen atoms or OM groups. The number of the oxygen atoms or OM groups of R²⁰¹ to R²¹⁰ may be an even number of 4 or larger. R²⁰¹ to R²¹⁰ may be all oxygen atoms or OM groups.

Each of R²⁰¹ to R²¹⁰ may have a configuration similar to each of R¹⁰¹ to R¹⁰⁸ described above. For example, R²⁰¹ to R²¹⁰ each independently represent an oxygen atom, a group represented by -OM, a hydrogen atom or an organic group. In addition, each of R²⁰¹ to R²¹⁰ may be determined such that the chemical compound represented by the general formula (2) has symmetry. Each of R²⁰¹ to R²¹⁰ may be determined such that the chemical compound represented by the general formula (2) has an axis of symmetry, a plane of symmetry, or a center of symmetry.

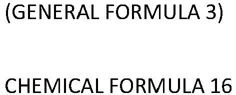

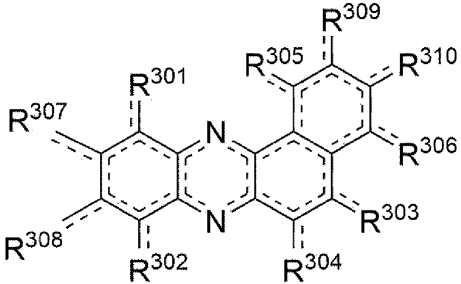

In the general formula (3), R³⁰¹ to R³¹⁰ each independently represent an oxygen atom, a group represented by -OM, a hydrogen atom or an organic group. M represents a hydrogen atom or a monovalent or bivalent metal atom. Note that, when M is a bivalent metal atom, two adjacent R³⁰¹ to R³¹⁰ are coupled to each other to form a ring represented by - OMO-. A double line represented by a solid line and a dashed line represents a single bond or a double bond.

In the present embodiment, at least four of R³⁰¹ to R³¹⁰ are oxygen atoms or OM groups. The number of the oxygen atoms or OM groups of R³⁰¹ to R³¹⁰ may be an even number of 4 or larger. R³⁰¹ to R³¹⁰ may be all oxygen atoms or OM groups.

Each of R³⁰¹ to R³¹⁰ may have a configuration similar to each of R¹⁰¹ to R¹⁰⁸ described above. For example, R³⁰¹ to R³¹⁰ each independently represent an oxygen atom, a group represented by -OM, a hydrogen atom or an organic group. In addition, each of R³⁰¹ to R³¹⁰ may be determined such that the chemical compound represented by the general formula (3) has symmetry. Each of R³⁰¹ to R³¹⁰ may be determined such that the chemical compound represented by the general formula (3) has an axis of symmetry, a plane of symmetry, or a center of symmetry.

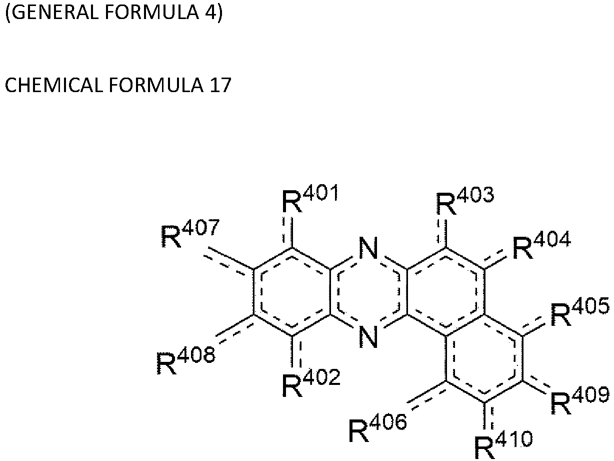

In the general formula (4), R⁴⁰¹ to R⁴¹⁰ each independently represent an oxygen atom, a group represented by -OM, a hydrogen atom or an organic group. M represents a hydrogen atom or a monovalent or bivalent metal atom. Note that, when M is a bivalent metal atom, two adjacent R⁴⁰¹ to R⁴¹⁰ are coupled to each other to form a ring represented by - OMO-. A double line represented by a solid line and a dashed line represents a single bond or a double bond.

In the present embodiment, at least four of R⁴⁰¹ to R⁴¹⁰ are oxygen atoms or OM groups. The number of the oxygen atoms or OM groups of R⁴⁰¹ to R⁴¹⁰ may be an even number of 4 or larger. R⁴⁰¹ to R⁴¹⁰ may be all oxygen atoms or OM groups.

Each of R⁴⁰¹ to R⁴¹⁰ may have a configuration similar to each of R¹⁰¹ to R¹⁰⁸ described above. For example, R⁴⁰¹ to R⁴¹⁰ each independently represent an oxygen atom, a group represented by -OM, a hydrogen atom or an organic group. In addition, each of R⁴⁰¹ to R⁴¹⁰ may be determined such that the chemical compound represented by the general formula (4) has symmetry. Each of R⁴⁰¹ to R⁴¹⁰ may be determined such that the chemical compound represented by the general formula (4) has an axis of symmetry, a plane of symmetry, or a center of symmetry.

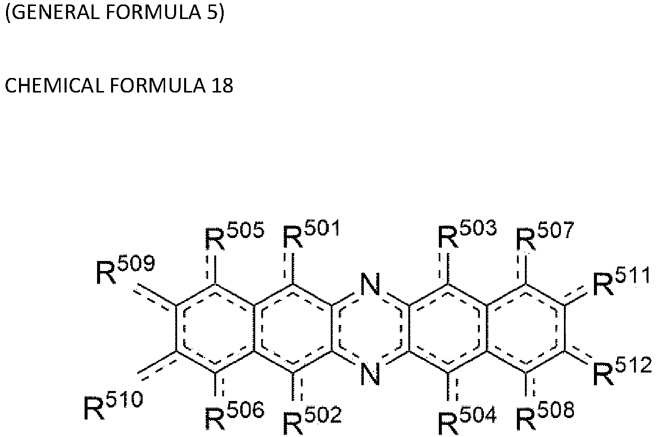

In the general formula (5), R⁵⁰¹ to R⁵¹² each independently represent an oxygen atom, a group represented by -OM, a hydrogen atom or an organic group. M represents a hydrogen atom or a monovalent or bivalent metal atom. Note that, when M is a bivalent metal atom, two adjacent R⁵⁰¹ to R⁵¹² are coupled to each other to form a ring represented by - OMO-. A double line represented by a solid line and a dashed line represents a single bond or a double bond.

In the present embodiment, at least four of R⁵⁰¹ to R⁵¹² are oxygen atoms or OM groups. The number of the oxygen atoms or OM groups of R⁵⁰¹ to R⁵¹² may be an even number of 4 or larger. R⁵⁰¹ to R⁵¹² may be all oxygen atoms or OM groups.

Each of R⁵⁰¹ to R⁵¹² may have a configuration similar to each of R¹⁰¹ to R¹⁰⁸ described above. For example, R⁵⁰¹ to R⁵¹² each independently represent an oxygen atom, a group represented by -OM, a hydrogen atom or an organic group. In addition, each of R⁵⁰¹ to R⁵¹² may be determined such that the chemical compound represented by the general formula (5) has symmetry. Each of R⁵⁰¹ to R⁵¹² may be determined such that the chemical compound represented by the general formula (5) has an axis of symmetry, a plane of symmetry, or a center of symmetry.

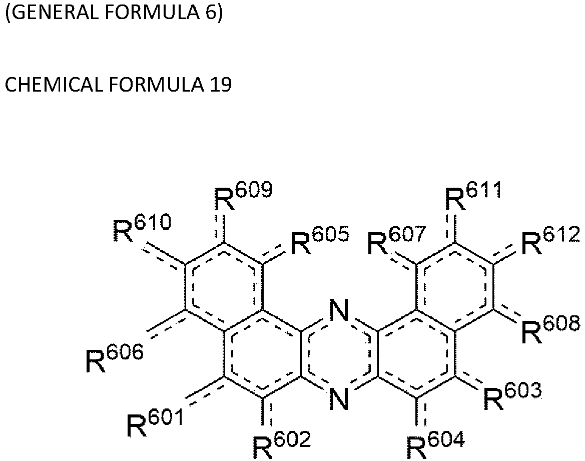

In the general formula (6), R⁶⁰¹ to R⁶¹² each independently represent an oxygen atom, a group represented by -OM, a hydrogen atom or an organic group. M represents a hydrogen atom or a monovalent or bivalent metal atom. Note that, when M is a bivalent metal atom, two adjacent R⁶⁰¹ to R⁶¹² are coupled to each other to form a ring represented by - OMO-. A double line represented by a solid line and a dashed line represents a single bond or a double bond.

In the present embodiment, at least four of R⁶⁰¹ to R⁶¹² are oxygen atoms or OM groups. The number of the oxygen atoms or OM groups of R⁶⁰¹ to R⁶¹² may be an even number of 4 or larger. R⁶⁰¹ to R⁶¹² may be all oxygen atoms or OM groups.

Each of R⁶⁰¹ to R⁶¹² may have a configuration similar to each of R¹⁰¹ to R¹⁰⁸ described above. For example, R⁶⁰¹ to R⁶¹² each independently represent an oxygen atom, a group represented by -OM, a hydrogen atom or an organic group. In addition, each of R⁶⁰¹ to R⁶¹² may be determined such that the chemical compound represented by the general formula (6) has symmetry. Each of R⁶⁰¹ to R⁶¹² may be determined such that the chemical compound represented by the general formula (6) has an axis of symmetry, a plane of symmetry, or a center of symmetry.

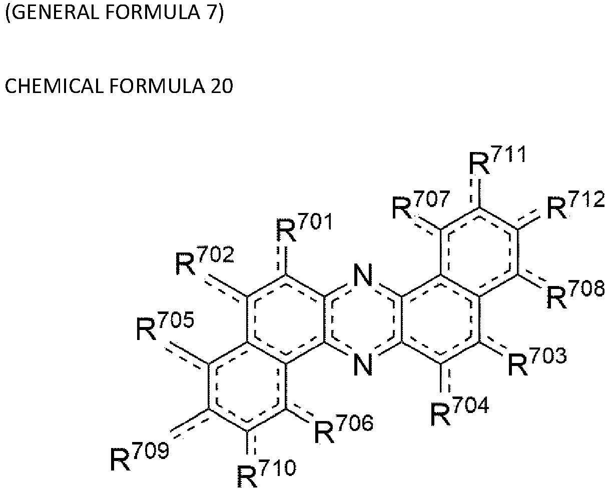

In the general formula (7), R⁷⁰¹ to R⁷¹² each independently represent an oxygen atom, a group represented by -OM, a hydrogen atom or an organic group. M represents a hydrogen atom or a monovalent or bivalent metal atom. Note that, when M is a bivalent metal atom, two adjacent R⁷⁰¹ to R⁷¹² are coupled to each other to form a ring represented by - OMO-. A double line represented by a solid line and a dashed line represents a single bond or a double bond.

In the present embodiment, at least four of R⁷⁰¹ to R⁷¹² are oxygen atoms or OM groups. The number of the oxygen atoms or OM groups of R⁷⁰¹ to R⁷¹² may be an even number of 4 or larger. R⁷⁰¹ to R⁷¹² may be all oxygen atoms or OM groups.

Each of R⁷⁰¹ to R⁷¹² may have a configuration similar to each of R¹⁰¹ to R¹⁰⁸ described above. For example, R⁷⁰¹ to R⁷¹² each independently represent an oxygen atom, a group represented by -OM, a hydrogen atom or an organic group. In addition, each of R⁷⁰¹ to R⁷¹² may be determined such that the chemical compound represented by the general formula (7) has symmetry. Each of R⁷⁰¹ to R⁷¹² may be determined such that the chemical compound represented by the general formula (7) has an axis of symmetry, a plane of symmetry, or a center of symmetry.

### (SPECIFIC EXAMPLES OF CHEMICAL COMPOUND USED AS ACTIVE MATERIAL)

In one embodiment, specific examples of the active material include the following chemical compounds. In the following chemical compounds, R¹, R², R³ and R⁴ each independently represent a hydrogen atom or an organic group. The organic groups represented by R¹ to R⁴ may have a configuration similar to the organic groups represented by R¹⁰¹ to R¹⁰⁸.

The following chemical compounds correspond to a state in which the active material is fully charged. The following chemical compounds may be examples of the fully oxidized form of the chemical compound represented by the general formula (1), general formula (2), general formula (3), general formula (4), general formula (4), general formula (5), general formula (6) or general formula (7). Note that the following chemical compounds are conjugated molecules and have resonance structures. The chemical compound represented by the general formula (1), general formula (2), general formula (3), general formula (4), general formula (4), general formula (5), general formula (6) or general formula (7) is not limited thereto. As another specific example, a fully reduced form or partial reduced form of the following chemical compounds may be exemplified.

### (EXAMPLE OF FULLY OXIDIZED FORM)

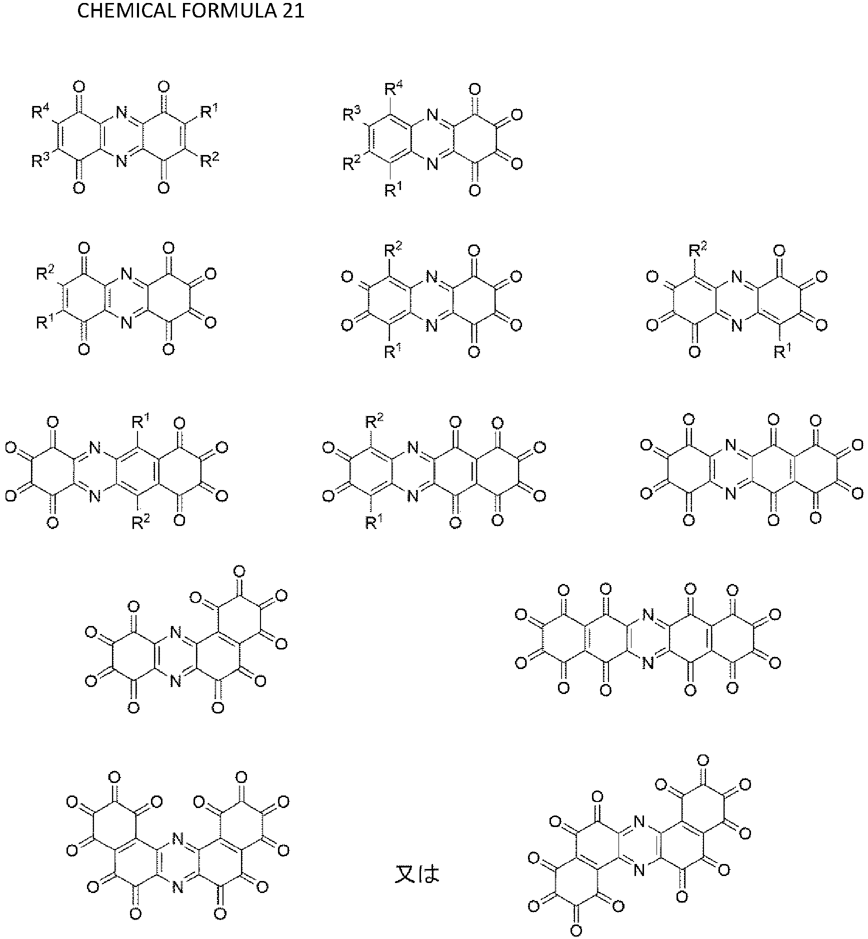

In another embodiment, the following chemical compounds are exemplified as specific examples of the active material. In the following chemical compounds, R⁵, R⁶, R⁷ and R⁸ each independently represent a hydrogen atom or an organic group. The organic groups represented by R⁵ to R⁸ may have a configuration similar to the organic groups represented by R¹⁰¹ to R¹⁰⁸.

The following chemical compounds correspond to a state in which the active material is completely discharged. The following chemical compounds may be examples of the fully reduced form of the chemical compound represented by the general formula (1), general formula (2), general formula (3), general formula (4), general formula (4), general formula (5), general formula (6) or general formula (7). The following chemical compounds may be fully reduced forms of the chemical compounds exemplified as chemical compounds corresponding to a state in which the active material is fully charged. Note that the following chemical compounds are conjugated molecules and have resonance structures.

### (EXAMPLE OF FULLY REDUCED FORM)

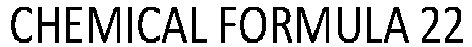

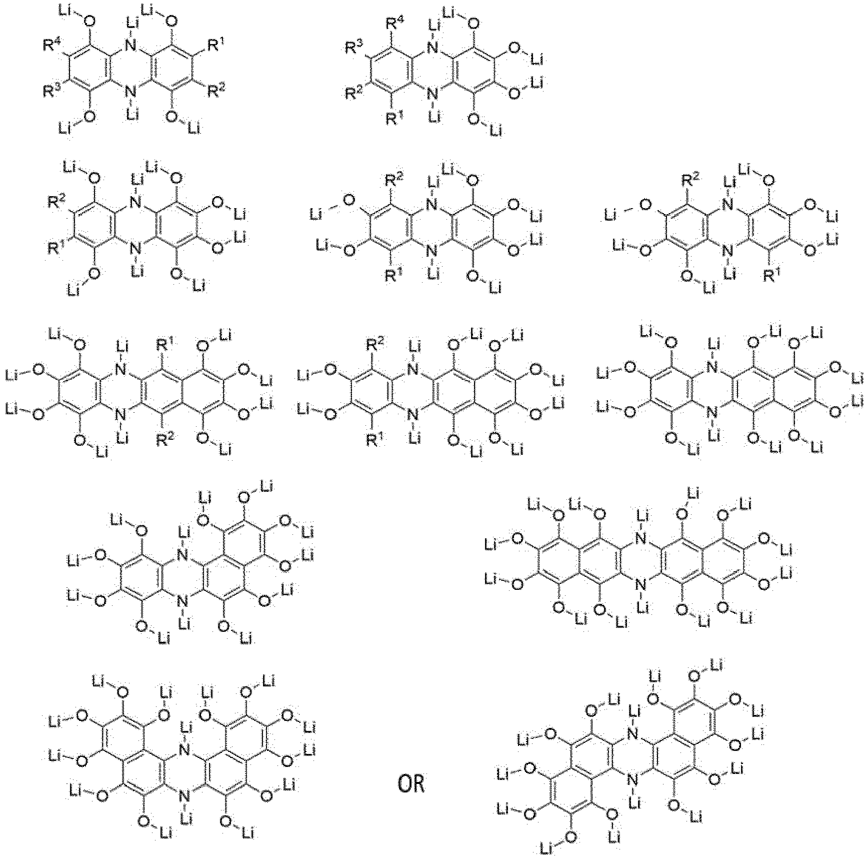

In the above, taking as an example the case where M in the general formulas (1) to (7) is Li, the chemical compounds corresponding to a state in which the active material is completely discharged have been exemplified. However, the M is not limited to Li. As described above, M may be a hydrogen atom or a monovalent or bivalent metal atom.

As is clear from the specific examples of the fully oxidized form and fully reduced form of the chemical compound, the fully reduced form or partial reduced form is different from the fully oxidized form, in that C=O of the 6-membered ring of carbon in the fully oxidized form becomes C-OM and =N- of the pyrazine ring in a fully oxidized form becomes -NM-. In addition, in addition, the fully reduced form or partial reduced form is different from the fully oxidized form, in that the 6-membered ring of carbon becomes an aromatic ring and the nitrogen-containing 6-membered ring becomes the structure shown in the chemical formula. The bond between the OMs in the C-OM may be an ionic bond. The bond between NMs in the NM may be an ionic bond.

### (SPECIFIC EXAMPLES OF CHEMICAL COMPOUND REPRESENTED BY GENERAL FORMULA (1))

In the chemical compound represented by the general formula (1), the ratio of the molecular weight of the chemical compound to the number of electrons involved in the oxidation-reduction reaction in the chemical compound is small. In addition, in the chemical compound represented by the general formula (1), when those other than an oxygen atom or OM group of R¹⁰¹ to R¹⁰⁸ are hydrogen atoms, the ratio of the molecular weight of the chemical compound to the number of electrons involved in the oxidation-reduction reaction in the chemical compound becomes smaller.

Below, a specific example of a fully oxidized form of the chemical compound represented by the general formula (1) is disclosed, taking as an example a case where four of R¹⁰¹ to R¹⁰⁸ are oxygen atoms or OM groups and those other than an oxygen atom or OM group of R¹⁰¹ to R¹⁰⁸
are hydrogen atoms. However, the chemical compound represented by the general formula (1) is not limited to thereto. As another specific example, a fully reduced form or partial reduced form of the following chemical compounds may be exemplified. Note that the following chemical compounds are conjugated molecules and have resonance structures.

### (SPECIFIC EXAMPLES OF FULLY OXIDIZED FORM)

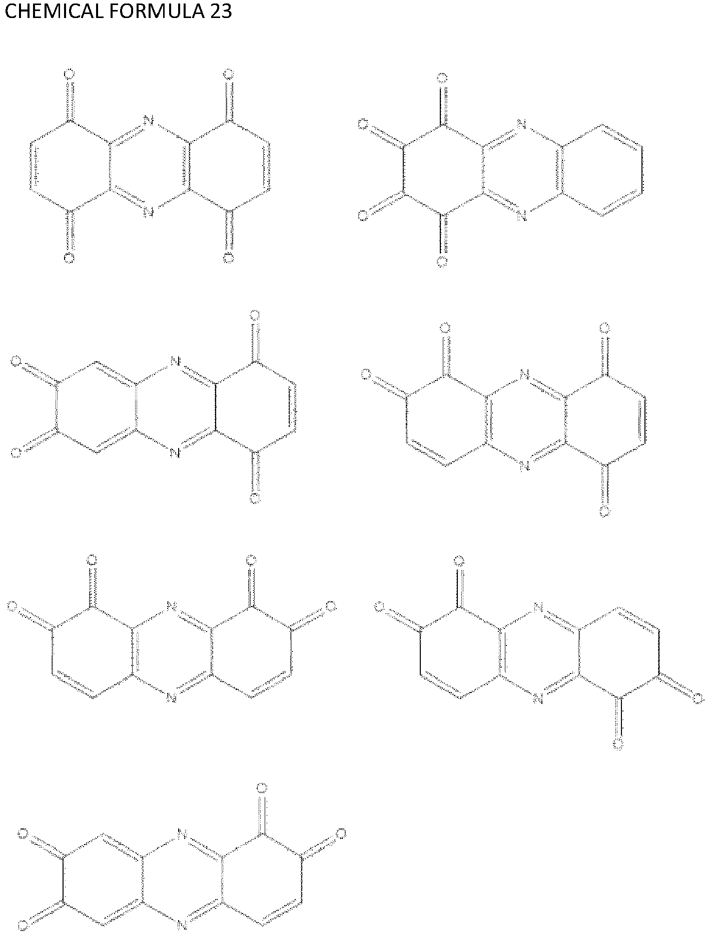

Below, a specific example of a fully oxidized form of the chemical compound represented by the general formula (1) is disclosed, taking as an example a case where six of R¹⁰¹ to R¹⁰⁸ are oxygen atoms or OM groups and those other than an oxygen atom or OM group of R¹⁰¹ to R¹⁰⁸
are hydrogen atoms. However, the chemical compound represented by the general formula (1) is not limited to thereto. As another specific example, a fully reduced form or partial reduced form of the following chemical compounds may be exemplified. Note that the following chemical compounds are conjugated molecules and have resonance structures.

### (SPECIFIC EXAMPLES OF FULLY OXIDIZED FORM)

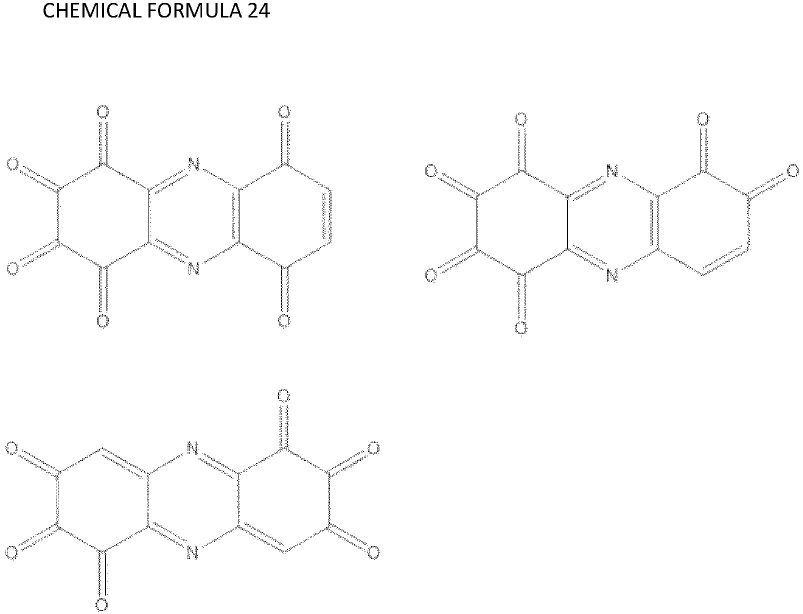

Below, a specific example of a fully oxidized form of the chemical compound represented by the general formula (1) is disclosed, taking as an example a case where all of R¹⁰¹ to R¹⁰⁸ are oxygen atoms or OM groups. However, the chemical compound represented by the general formula (1) is not limited to thereto. As another specific example, a fully reduced form or partial reduced form of the following chemical compounds may be exemplified. Note that the following chemical compounds are conjugated molecules and have resonance structures.

### (SPECIFIC EXAMPLES OF FULLY OXIDIZED FORM)

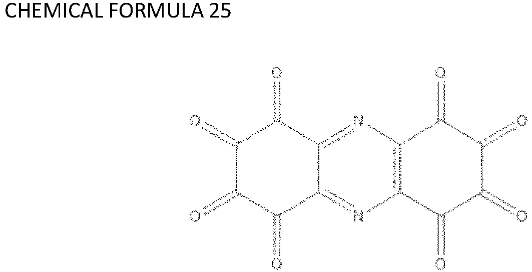

### (SPECIFIC EXAMPLES OF CHEMICAL COMPOUND REPRESENTED BY GENERAL FORMULA (2))

In the chemical compound represented by the general formula (2), the ratio of the molecular weight of the chemical compound to the number of electrons involved in the oxidation-reduction reaction in the chemical compound is small. In addition, in the chemical compound represented by the general formula (2), when those other than an oxygen atom or OM group of R²⁰¹ to R²¹⁰
are hydrogen atoms, the ratio of the molecular weight of the chemical compound to the number of electrons involved in the oxidation-reduction reaction in the chemical compound becomes smaller.

Below, a specific example of a fully oxidized form of the chemical compound represented by the general formula (2) is disclosed, taking as an example a case where four of R²⁰¹ to R²¹⁰ are oxygen atoms or OM groups and those other than an oxygen atom or OM group of R²⁰¹ to R²¹⁰ are hydrogen atoms. However, the chemical compound represented by the general formula (2) is not limited to thereto. As another specific example, a fully reduced form or partial reduced form of the following chemical compounds may be exemplified. Note that the following chemical compounds are conjugated molecules and have resonance structures.

### (SPECIFIC EXAMPLES OF FULLY OXIDIZED FORM)

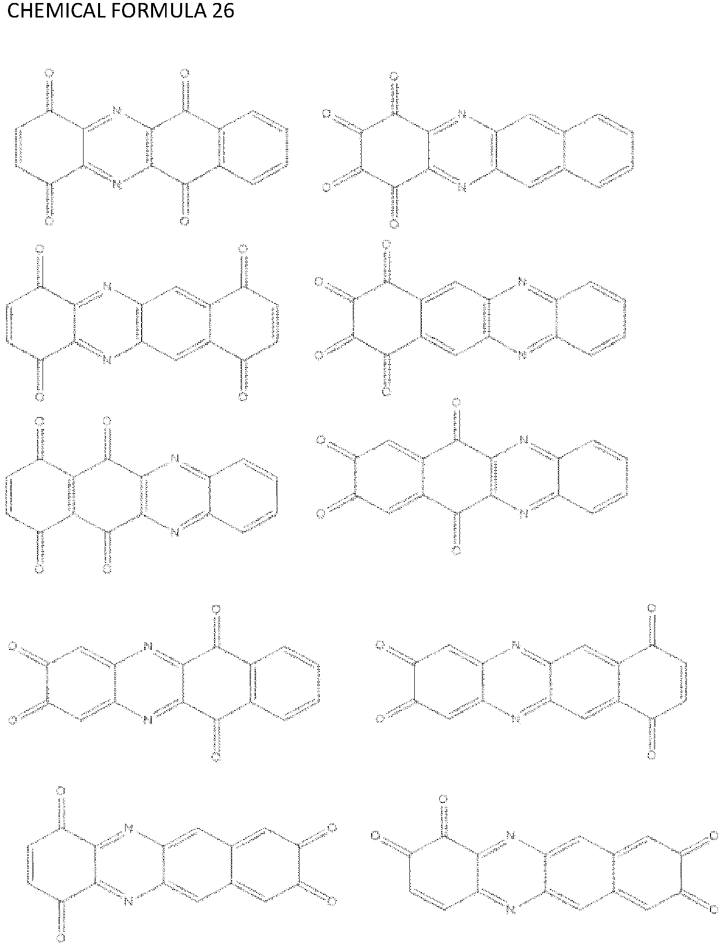

Below, a specific example of a fully oxidized form of the chemical compound represented by the general formula (2) is disclosed, taking as an example a case where six of R²⁰¹ to R²¹⁰ are oxygen atoms or OM groups and those other than an oxygen atom or OM group of R²⁰¹ to R²¹⁰ are hydrogen atoms. However, the chemical compound represented by the general formula (2) is not limited to thereto. As another specific example, a fully reduced form or partial reduced form of the following chemical compounds may be exemplified. Note that the following chemical compounds are conjugated molecules and have resonance structures.

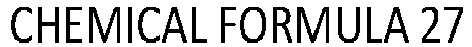

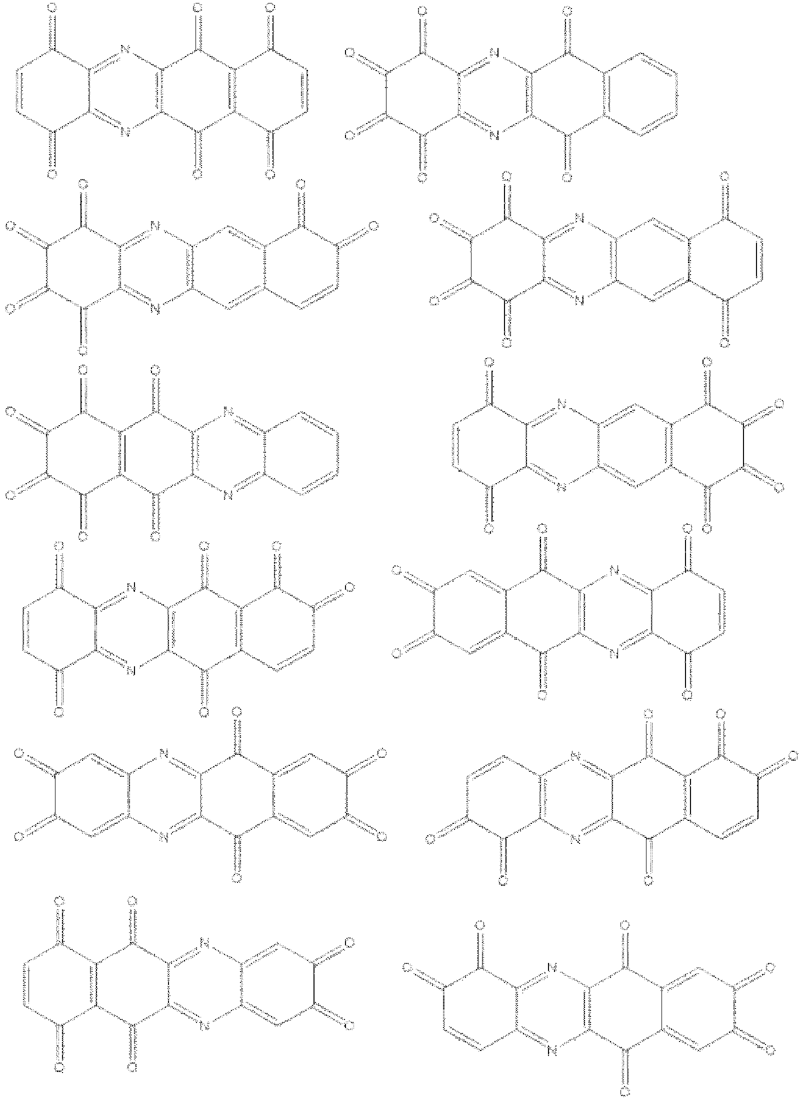

Below, a specific example of a fully oxidized form of the chemical compound represented by the general formula (2) is disclosed, taking as an example a case where eight of R²⁰¹ to R²¹⁰ are oxygen atoms or OM groups and those other than an oxygen atom or OM group of R²⁰¹ to R²¹⁰
are hydrogen atoms. However, the chemical compound represented by the general formula (2) is not limited to thereto. As another specific example, a fully reduced form or partial reduced form of the following chemical compounds may be exemplified. Note that the following chemical compounds are conjugated molecules and have resonance structures.

### (SPECIFIC EXAMPLES OF FULLY OXIDIZED FORM)

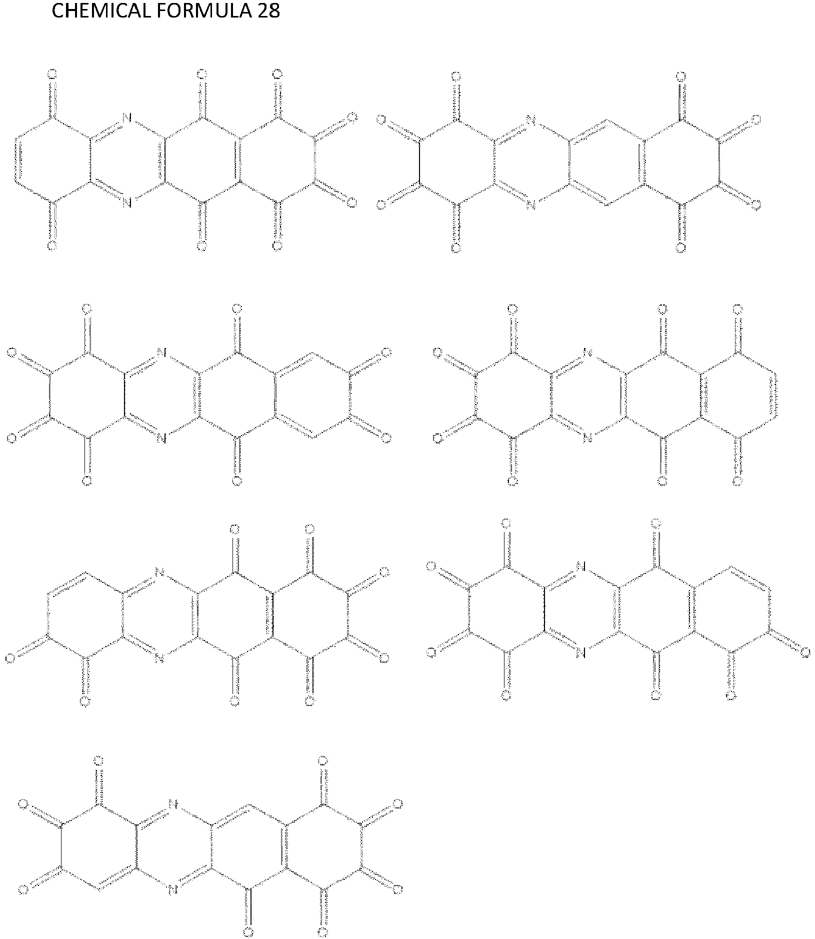

Below, a specific example of a fully oxidized form of the chemical compound represented by the general formula (2) is disclosed, taking as an example a case where ten of R²⁰¹ to R²¹⁰ are oxygen atoms or OM groups. Also, a specific example of a fully oxidized form of the chemical compound represented by the general formula (3) is disclosed, taking as an example a case where ten of R³⁰¹ to R³¹⁰ are oxygen atoms or OM groups. However, the chemical compound represented by the general formula (2) or general Formula (3) is not limited thereto. As another specific example, a fully reduced form or partial reduced form of the following chemical compounds may be exemplified. Note that the following chemical compounds are conjugated molecules and have resonance structures.

### (SPECIFIC EXAMPLES OF FULLY OXIDIZED FORM)

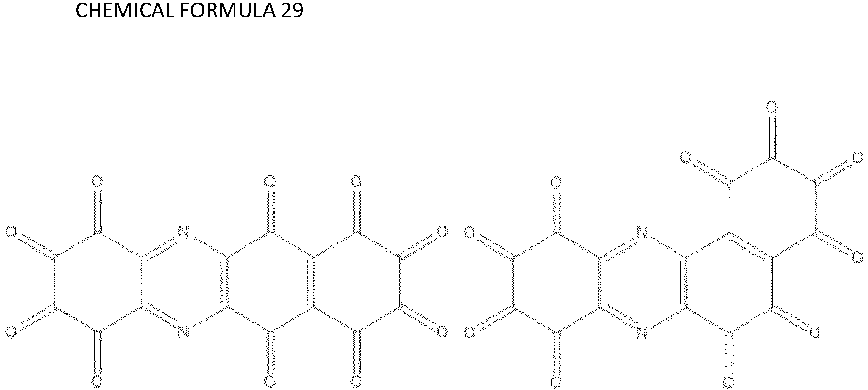

Below, a specific example of a fully oxidized form of the chemical compound represented by the general formula (3) is disclosed, taking as an example a case where six of R³⁰¹ to R³¹⁰ are oxygen atoms or OM groups and those other than an oxygen atom or OM group of R³⁰¹ to R³¹⁰
are hydrogen atoms. However, the chemical compound represented by the general formula (3) is not limited to thereto. As another specific example, a fully reduced form or partial reduced form of the following chemical compounds may be exemplified. Note that the following chemical compounds are conjugated molecules and have resonance structures.

### (SPECIFIC EXAMPLES OF FULLY OXIDIZED FORM)

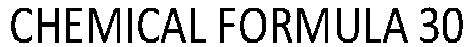

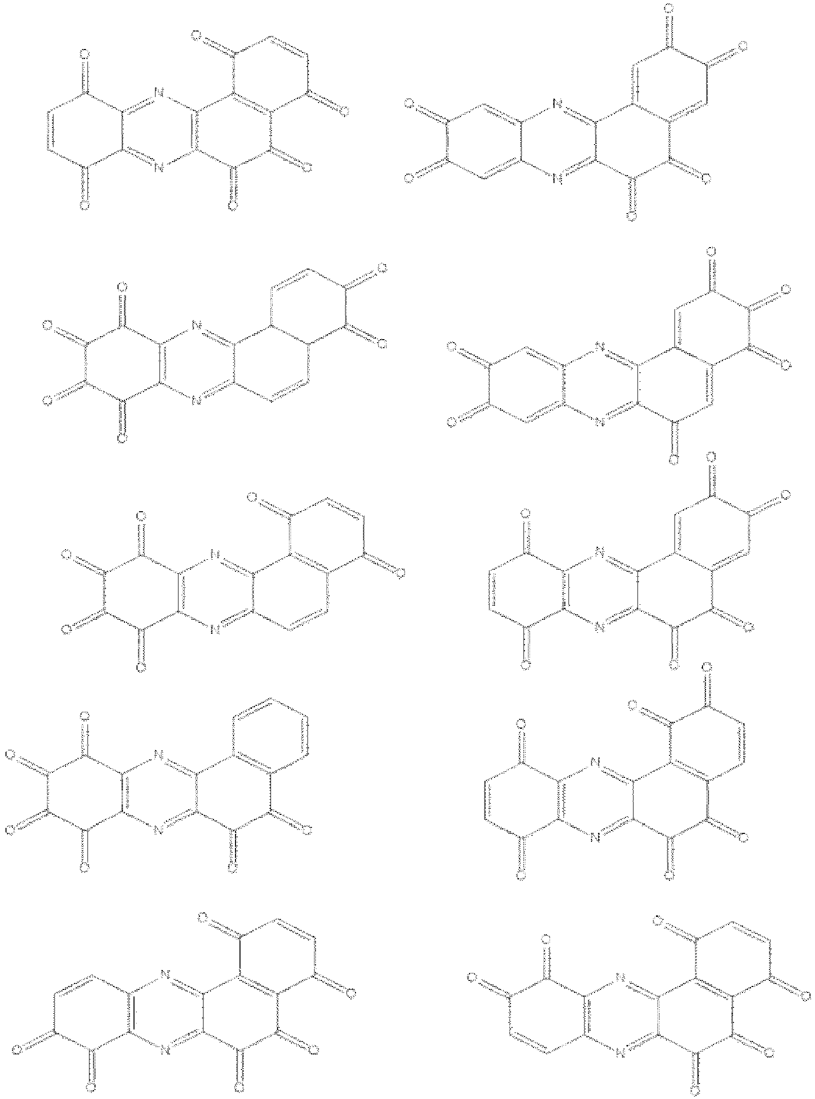

Below, a specific example of a fully oxidized form of the chemical compound represented by the general formula (3) is disclosed, taking as an example a case where eight of R³⁰¹ to R³¹⁰ are oxygen atoms or OM groups and those other than an oxygen atom or OM group of R³⁰¹ to R³¹⁰
are hydrogen atoms. However, the chemical compound represented by the general formula (3) is not limited to thereto. As another specific example, a fully reduced form or partial reduced form of the following chemical compounds may be exemplified. Note that the following chemical compounds are conjugated molecules and have resonance structures.

### (SPECIFIC EXAMPLES OF FULLY OXIDIZED FORM)

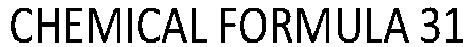

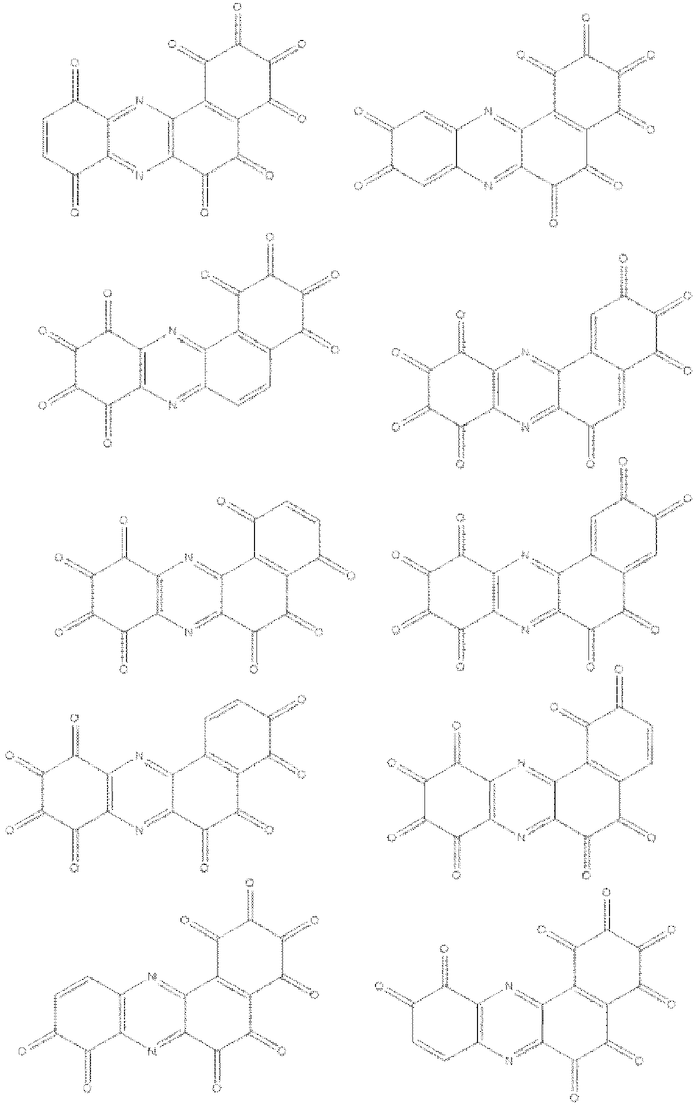

### (SPECIFIC EXAMPLES OF CHEMICAL COMPOUND HAVING CENTER OF SYMMETRY)

As described above, the active material described above preferably includes a chemical compound having a small molecular weight and symmetry among the chemical compounds represented by the general formula (1) to the general formula (7). The active material may be a chemical compound represented by the following chemical formula (1A-1) or chemical formula (1B-1). In the chemical formula (1B-1), M represents a hydrogen atom or a monovalent metal atom. M may be H, Li, Na or K.

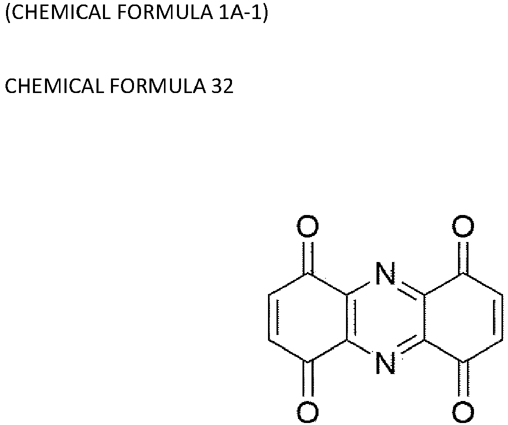

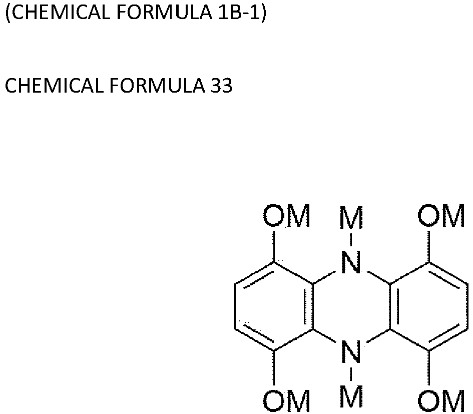

The chemical formula (1A-1) corresponds to a state in which the active material is fully charged. The chemical formula (1B-1) corresponds to a state in which the active material is completely discharged. For example, when the storage cell 112 is a lithium ion secondary battery or a lithium metal secondary battery, the battery electrode reaction is represented by the following reaction formula 1.

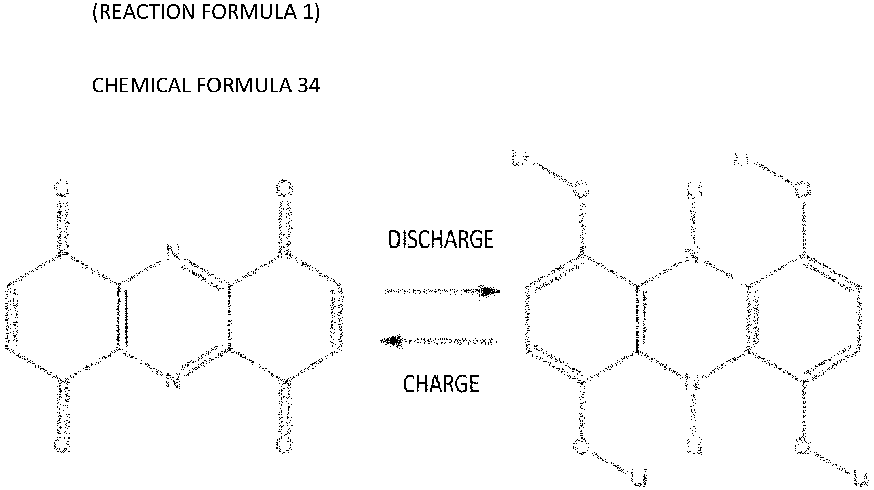

That is, in a state in which the active material is fully charged, all of four oxygens bonded to the benzene ring included in the phenazine structure become oxygen atoms, and the chemical compound represented by the chemical formula (1A-1) can be formed. Thereafter, as the discharging of the active material proceeds, lithium atoms are intercalated into some of the oxygen atoms. Then, in a state in which the active material is completely discharged, all of four oxygens bonded to the benzene ring included in the phenazine structure combine with Li, and the chemical compound represented by the chemical formula (1B-1) can be formed.

In the reaction formula 1, the number of moving electrons is 6, and the theoretical capacity of the chemical compound represented by the chemical formula (1A-2) or chemical formula (1B-2) is 670 [mAh/g]. The theoretical capacity [mAh/g]is calculated by 26800 ÷ molar mass [g/mol]x number of moving electrons [number].

The active material may be a chemical compound represented by the following chemical formula (1A-2) or chemical formula (1B-2). In the chemical formula (1B-2), M represents a hydrogen atom or a monovalent metal atom. M may be H, Li, Na or K.

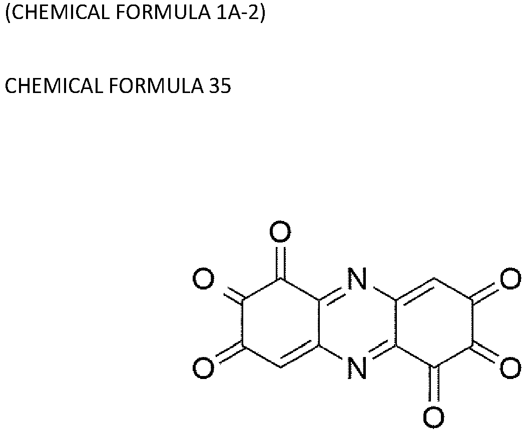

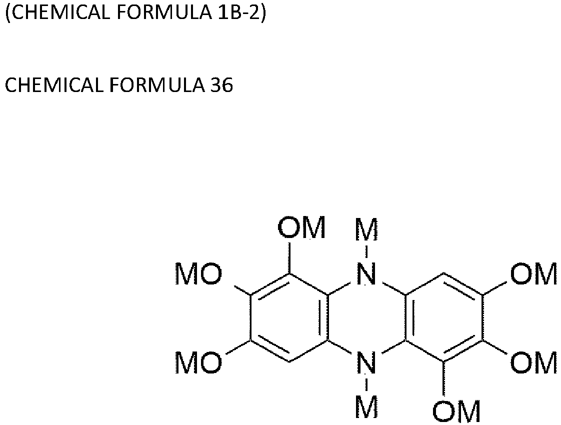

The chemical formula (1A-2) corresponds to a state in which the active material is fully charged. The chemical formula (1B-2) corresponds to a state in which the active material is completely discharged. For example, when the storage cell 112 is a lithium ion secondary battery or a lithium metal secondary battery, the battery electrode reaction is represented by the following reaction formula 2.

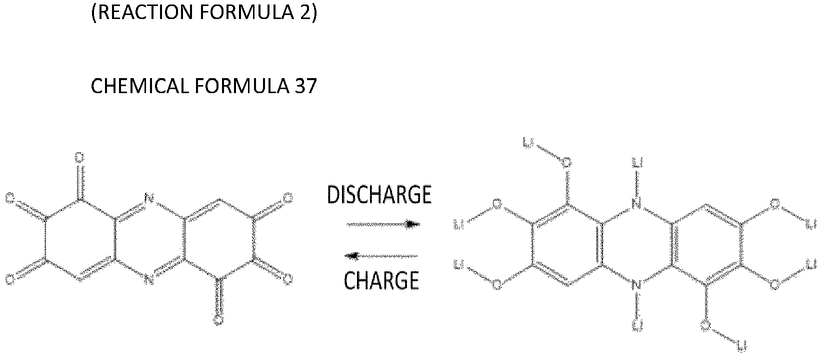

That is, in a state in which the active material is completely charged, all of six oxygens bonded to the benzene ring included in the phenazine structure become oxygen atoms, and the chemical compound represented by the chemical formula (1A-2) can be formed. Thereafter, as the discharging of the active material proceeds, lithium atoms are intercalated into some of the oxygen atoms. Then, in a state in which the active material is completely discharged, all of six oxygens bonded to the benzene ring included in the phenazine structure combine with Li, and the chemical compound represented by the chemical formula (1B-2) can be formed. In the reaction formula 2, the number of moving electrons is 8, and the theoretical capacity of the chemical compound represented by the chemical formula (1A-2) or chemical formula (1B-2) is 777 [mAh/g].

The active material may be a chemical compound represented by the following chemical formula (1A-3) or chemical formula (1B-3). In the chemical formula (1B-3), M represents a hydrogen atom or a monovalent metal atom. M may be H, Li, Na or K.

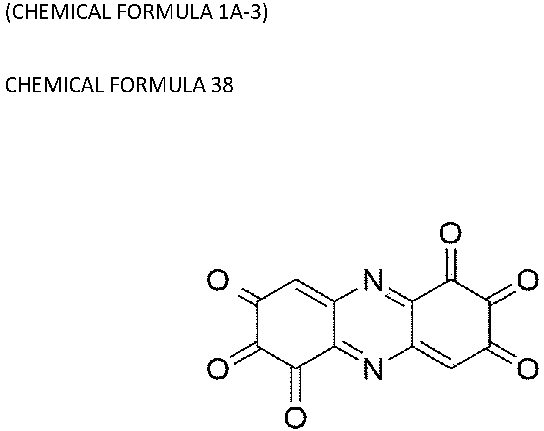

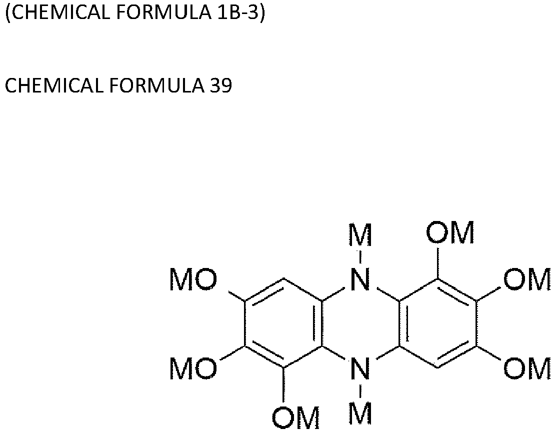

The chemical formula (1A-3) corresponds to a state in which the active material is fully charged. The chemical formula (1B-3) corresponds to a state in which the active material is completely discharged.

The active material may be a chemical compound represented by the following chemical formula (1A-4) or chemical formula (1B-4). In the chemical formula (1B-4), M represents a hydrogen atom or a monovalent metal atom. M may be H, Li, Na or K.

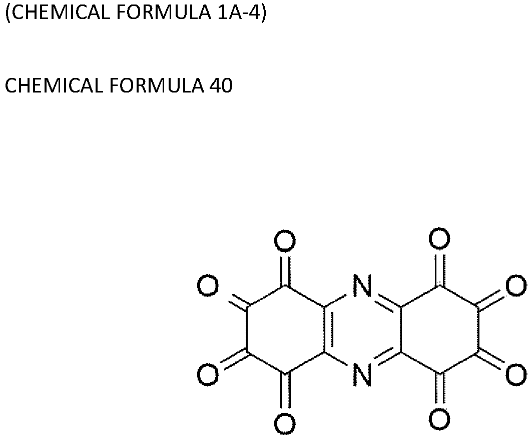

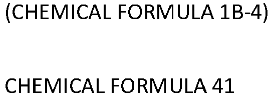

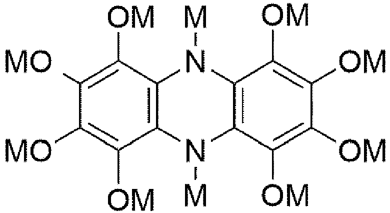

The chemical formula (1A-4) corresponds to a state in which the active material is fully charged. The chemical formula (1B-4) corresponds to a state in which the active material is completely discharged. For example, when the storage cell 112 is a lithium ion secondary battery or a lithium metal secondary battery, the battery electrode reaction is represented by the following reaction formula 3.

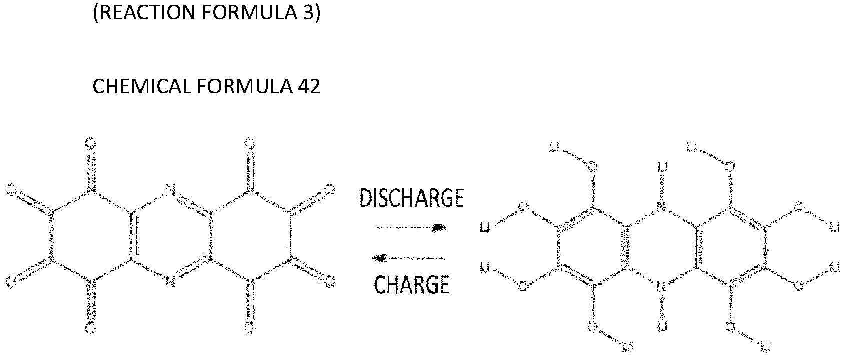

That is, in a state in which the active material is completely charged, all of eight oxygens bonded to the benzene ring included in the phenazine structure become oxygen atoms, and the chemical compound represented by the chemical formula (1A-3) can be formed. Thereafter, as the discharging of the active material proceeds, lithium atoms are intercalated into some of the oxygen atoms. Then, in a state in which the active material is completely discharged, all of eight oxygens bonded to the benzene ring included in the phenazine structure combine with Li, and the chemical compound represented by the chemical formula (1B-3) can be formed. In the reaction formula 3, the number of moving electrons is 10, and the theoretical capacities of the chemical compounds represented by the chemical formula (1A-3) and chemical formula (1B-3) are each 893 [mAh/g]and 726 [mAh/g].

As described above, the chemical compounds represented by the chemical formula (1A-1), the chemical formula (1A-2) and the chemical formula (1A-3) have, for example, a lithium atom intercalated into an oxygen atom to have a group represented by -OLi. Therefore, these chemical compounds can be used as the positive electrode active material. Note that, among the chemical compounds represented by the general formula (1) to the general formula (8), a chemical compound in which a group bonded to the benzene ring included in the phenazine structure (for example, in the case of the chemical compound represented by the general formula (1), groups represented by R¹⁰¹ to R¹⁰⁸) is an oxygen atom is also the same.

Similarly, in the chemical compounds represented by the chemical formula (1B-1), the chemical formula (1B-2) and the chemical formula (1B-3), for example, a lithium atom is deintercalated from the group represented by -OLi to leave an oxygen atom. Therefore, these chemical compounds can be used as a negative electrode active material. Note that, among the chemical compounds represented by the general formula (1) to the general formula (8), a chemical compound in which a group bonded to the benzene ring included in the phenazine structure (for example, in the case of the chemical compound represented by the general formula (1), groups represented by R¹⁰¹ to R¹⁰⁸) is an OM group is also the same.

### (SECOND EMBODIMENT)

In another embodiment, the active material includes a polymer including a repeating unit represented by the following general formula (8), general formula (9), general formula (10) or general formula (11), or a salt thereof. The polymer or salt thereof may be included in any of a reaction starting material, a product and an intermediate product in a discharging reaction of a battery electrode reaction. The polymer or salt thereof may be included in any of a reaction starting material, a product and an intermediate product in a charging reaction of the battery electrode reaction.

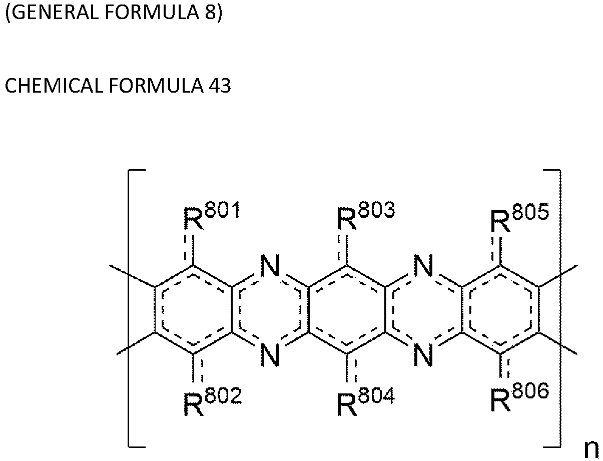

In the general formula (8), R⁸⁰¹ to R⁸⁰⁶ each independently represent an oxygen atom, a group represented by -OM, a hydrogen atom or an organic group. M represents a hydrogen atom or a monovalent or bivalent metal atom. Note that, when M is a bivalent metal atom, two adjacent R⁸⁰¹ to R⁸⁰⁶ are coupled to each other to form a ring represented by - OMO-. A double line represented by a solid line and a dashed line represents a single bond or a double bond. N is an integer of 2 or larger representing a polymerization degree.

In the present embodiment, at least four of R⁸⁰¹ to R⁸⁰⁶ are oxygen atoms or OM groups. The number of the oxygen atoms or OM groups of R⁸⁰¹ to R⁸⁰⁶ may be an even number of 4 or larger. R⁸⁰¹ to R⁸⁰⁶ may be all oxygen atoms or OM groups.

Each of R⁸⁰¹ to R⁸⁰⁶ may have a configuration similar to each of R¹⁰¹ to R¹⁰⁸ described above. For example, R⁸⁰¹ to R⁸⁰⁶ each independently represent an oxygen atom, a group represented by -OM, a hydrogen atom or an organic group. In addition, each of R⁸⁰¹ to R⁸⁰⁶ may be determined such that the repeating unit represented by the general formula (8) has symmetry. Each of R⁸⁰¹ to R⁸⁰⁶ may be determined such that the repeating unit represented by the general formula (8) has an axis of symmetry, a plane of symmetry, or a center of symmetry.

According to the present embodiment, since a storage site is included in the repeating unit, even when the polymerization degree and molecular weight of the polymer increase, the amount of electric charges that can be accumulated per unit mass does not fluctuate. For this reason, any polymerization degree n may be employed.

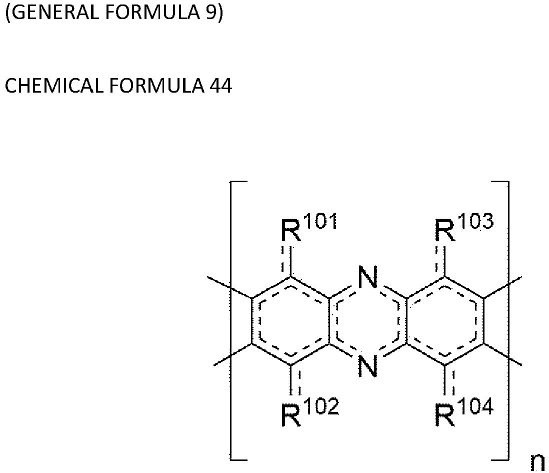

In the general formula (9), R¹⁰¹ to R¹⁰⁴ each independently represent an oxygen atom or a group represented by -OM. M represents a hydrogen atom or a monovalent or bivalent metal atom. Note that, when M is a bivalent metal atom, two adjacent R101 to R104 are coupled to each other to form a ring represented by -OMO-. A double line represented by a solid line and a dashed line represents a single bond or a double bond. N is an integer of 2 or larger representing a polymerization degree. The details of R¹⁰¹ to R¹⁰⁴ may be as described in relation to the general formula (1).

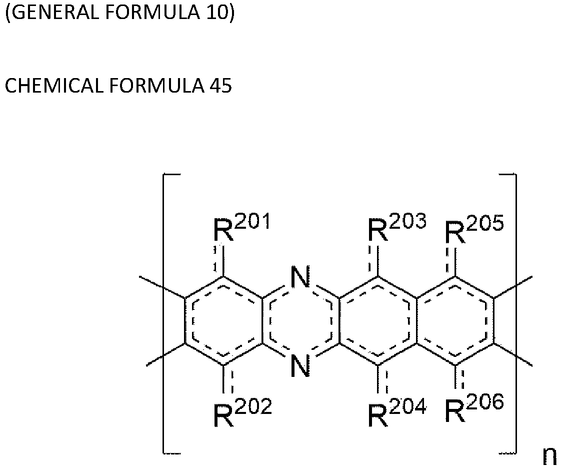

In the general formula (10), R²⁰¹ to R¹⁰⁶ each independently represent an oxygen atom, a group represented by -OM, a hydrogen atom or an organic group. M represents a hydrogen atom or a monovalent or bivalent metal atom. Note that, when M is a bivalent metal atom, two adjacent R²⁰¹ to R²⁰⁶ are coupled to each other to form a ring represented by - OMO-. A double line represented by a solid line and a dashed line represents a single bond or a double bond. At least four of R²⁰¹ to R²⁰⁶ are oxygen atoms, or groups represented by - OM. N is an integer of 2 or larger representing a polymerization degree. The details of R²⁰¹ to R²⁰⁶ may be as described in relation to the general formula (2).

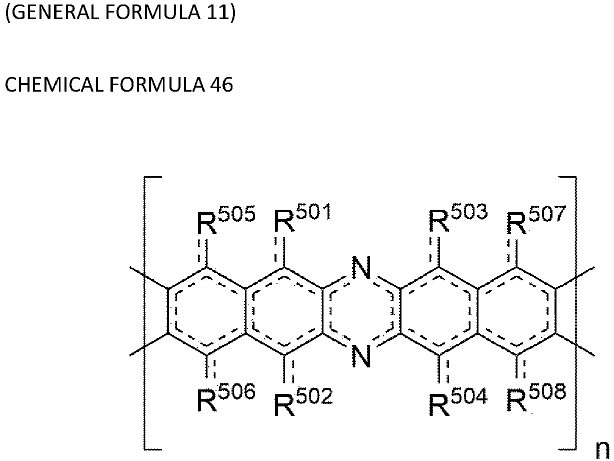

In the general formula (11), R⁵⁰¹ to R⁵⁰⁸ each independently represent an oxygen atom, a group represented by -OM, a hydrogen atom or an organic group. M represents a hydrogen atom or a monovalent or bivalent metal atom. Note that, when M is a bivalent metal atom, two adjacent R⁵⁰¹ to R⁵⁰⁸ are coupled to each other to form a ring represented by - OMO-. A double line represented by a solid line and a dashed line represents a single bond or a double bond. At least four of R⁵⁰¹ to R⁵⁰⁸ are oxygen atoms, or groups represented by - OM. N is an integer of 2 or larger representing a polymerization degree. The details of R⁵⁰¹ to R⁵⁰⁸ may be as described in relation to the general formula (5).

### (SPECIFIC EXAMPLES OF CHEMICAL COMPOUND USED AS ACTIVE MATERIAL)

The active material may also include a polymer including a repeating unit represented by the following chemical formula (8-1), or a salt thereof. In the chemical formula (8-1), n is an integer of 2 or larger representing the polymerization degree.

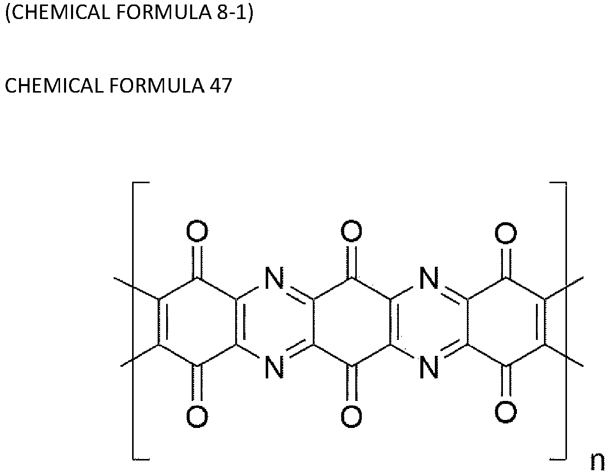

The polymer including the repeating unit represented by the chemical formula (8-1) corresponds to a state in which the active material is completely charged. The polymer including the repeating unit represented by the chemical formula (8-1) may be an example of a fully oxidized form of the polymer including the repeating unit represented by the general formula (8). Note that the following chemical compounds are conjugated molecules and have resonance structures. The polymer including the repeating unit represented by the general formula (8) or a salt thereof is not limited to the polymer including the repeating unit represented by the chemical formula (8-1). As another specific example, a fully reduced form or partial reduced form of the polymer including the repeating unit represented by the chemical formula (8-1) can be exemplified.

### (II. MANUFACTURING METHOD OF ACTIVE MATERIAL)

The active material described above may be a known chemical compound or can be synthesized by employing a known reaction. For example, phenazine-1,4,6,9-tetraone represented by the chemical formula (1A-1) can be synthesized according to the following reaction formula 4.

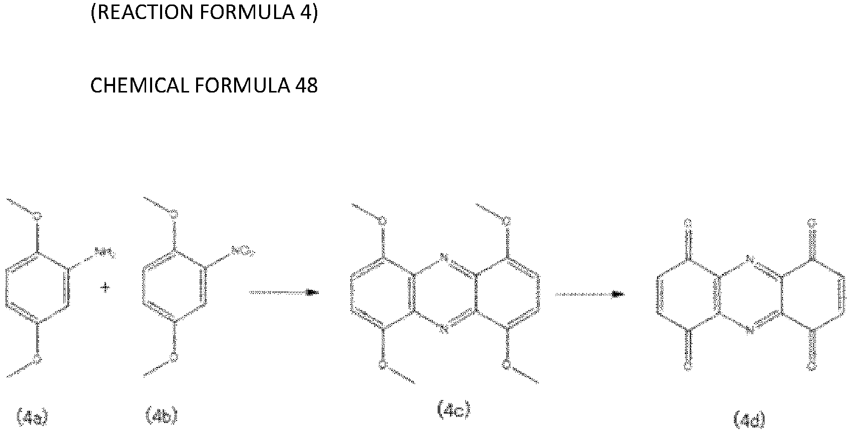

For example, first, 1,4,6,9-tetramethoxyphenazine (4c in the reaction formula 4) is obtained by a known method using 2,5-dimethoxyaniline (4a in the reaction formula 4) and 1,4-dimethoxy-2-nitrobenzene (4b in the reaction formula 4) as starting materials. For example, 1,4,6,9-tetramethoxyphenazine is produced by dissolving the starting materials in a toluene solvent and refluxing the same under presence of potassium tert-butoxide (t-BuOK).

Next, phenazine-1,4,6,9-tetraone (4d in the reaction formula 4) is obtained by a known method using 1,4,6,9-tetramethoxyphenazine as a starting material. For example, phenazine-1,4,6,9-tetraone is produced by mixing a solution obtained by dissolving 1,4,6,9-tetramethoxyphenazine in an organic solvent, and an aqueous solution of ammonium hexanitratocerate (IV).

In addition, a reduced form of phenazine-1,2,3,6,7,8-hexaone represented by the chemical formula (1A-3) can be synthesized according to the following reaction formula 5 and reaction formula 6. The reduced form of phenazine-1,2,3,6,7,8-hexaone may be an example of the chemical compound represented by the chemical formula (1B-3).

Similarly, a partial reduced form of phenazine-1,2,3,4,6,7,8,9-octaone represented by the chemical formula (1A-4) can be synthesized by the following reaction formula 5 and reaction formula 7. The partial reduced form of phenazine-1,2,3,4,6,7,8,9-octaone may be an example of the chemical compound represented by the chemical formula (1B-4).

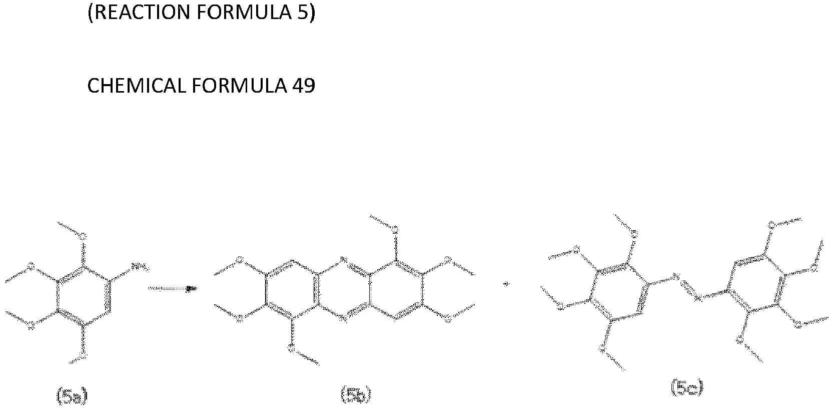

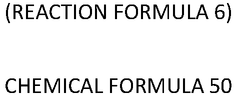

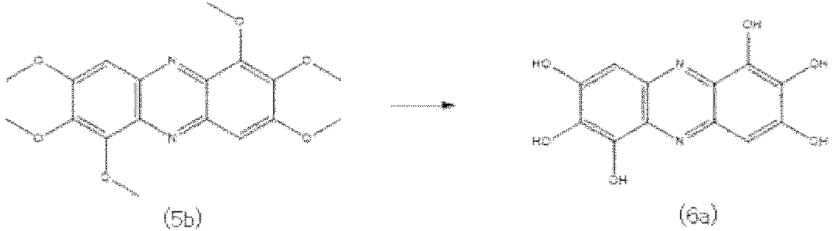

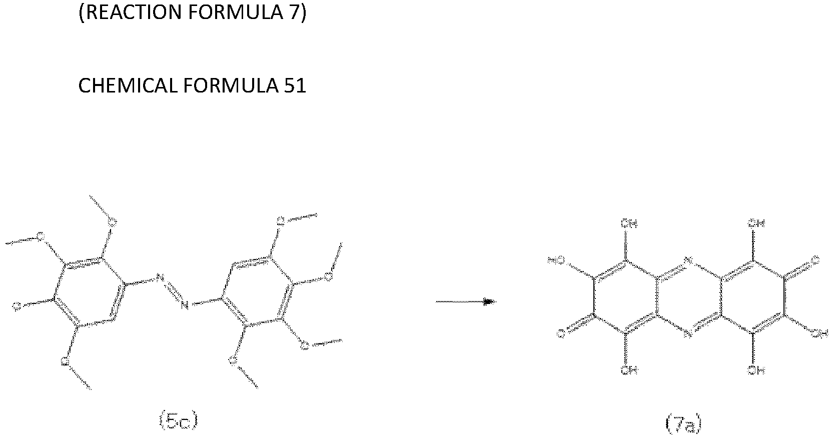

For example, first, in the reaction formula 5, 1,2,3,6,7,8-hexamethoxyphenazine (5b in the reaction formula 5) and (E)-1,2-bis(2,3,4,5-tetramethoxyphenyl) diazene (5c in the reaction formula 5) are obtained by a known method using 2,3,4,5-tetramethoxyaniline (5a in the reaction formula 5) as a starting material. For example, 1,2,3,6,7,8-hexamethoxyphenazine and (E)-1,2-bis(2,3,4,5-tetramethoxyphenyl) diazene are produced by dissolving the starting material in N,N-dimethylformamide (DMF) and heating and stirring the same with a known catalyst.

Next, in the reaction formula 6, 1,2,3,6,7,8-hexahydroxyphenazine (6a in the reaction formula 6) is obtained by a known method using, as a starting material, 1,2,3,6,7,8-hexamethoxyphenazine (5b in the reaction formula 6) obtained by the reaction formula 5. For example, 1,2,3,6,7,8-hexahydroxyphenazine is produced by dissolving the starting material in a mixed solution of acetic acid-hydrobromic acid and heating and stirring the same at a reflux temperature.

Similarly, in the reaction formula 7, 1,3,4,6,8,9-hexahydroxyphenazine-2,7-dione (7a in the reaction formula 7) is obtained by a known method using, as a starting material, (E)-1,2-bis(2,3,4,5-tetramethoxyphenyl) diazene (5c in the reaction formula 7) obtained by the reaction formula 5. For example, 1,3,4,6,8,9-hexahydroxyphenazine-2,7-dione is produced by dissolving the starting material in a mixed solution of acetic acid-hydrobromic acid and heating and stirring the same at a reflux temperature.

Note that one skilled in the art upon the reference to the description of the present specification can understand that the chemical compounds represented by the general formulas (1) to (7) and the repeating units represented by the general formulas (8) to (11) can be synthesized through similar procedures. In addition, one skilled in the art upon the reference to the description of the present specification can understand that the chemical compounds represented by the general formulas (1) to (7), the repeating units represented by the general formulas (8) to (11) and the polymers including the repeating units represented by the general formulas (8) to (11) can be synthesized by adopting known reactions, if necessary.

### EXAMPLES

### (EXAMPLE 1)

Phenazine-1,4,6,9-tetraone was synthesized according to the procedure described in Conboy, Darren; Mirallai, Styliana I.; Craig, Austin; McArdle, Patrick; Al-Kinani, Ali A.; Barton, Stephen; Aldabbagh, Fawaz, Journal of Organic Chemistry, 2019, vol.84, #15, p.9811-9818. The details of the reaction conditions were determined based on the above-described literature. In addition, commercially available reagents were used for various reagents used in the synthesis.

Specifically, first, 2,5-dimethoxyaniline and 1,4-dimethoxy-2-nitrobenzene were refluxed in a toluene solvent for 16 hours under presence of t-BuOK. The obtained solid content was separated by column chromatography to obtain 1,4,6,9-tetramethoxyphenazine.

Next, the separated 1,4,6,9-tetramethoxyphenazine was dissolved in an appropriate solvent, mixed with an aqueous solution of ammonium hexanitratocerate (IV), and stirred at room temperature for one hour. Thereafter, phenazine-1,4,6,9-tetraone was obtained by washing the organic layer with water by a liquid separation operation, and distilling off the solvent.

### (EXAMPLE 2)

1,2,3,6,7,8-hexahydroxyphenazine, which is a reduced form of phenazine-1,2,3,6,7,8-hexaone, was synthesized according to the procedure described in Chem. Commun., 2016, 52, 922-925. The details of the reaction conditions were determined based on the above-described literature. In addition, commercially available reagents were used for various reagents used in the synthesis.

Specifically, first, 2,3,4,5-tetramethoxyaniline was dissolved in DMF, which was then heated and stirred together with an appropriate catalyst. Then, 1,2,3,6,7,8-hexamethoxyphenazine and (E)-1,2-bis (2,3,4,5-tetramethoxyphenyl) diazene were obtained by a separation operation using column chromatography.

Next, the separated 1,2,3,6,7,8-hexamethoxyphenazine was dissolved in a mixed solution of an acetic acid-hydrobromic acid, which was then heated and stirred at a reflux temperature. Then, after cooling the solution to the room temperature, 1,2,3,6,7,8-hexahydroxyphenazine was obtained by a filtration operation.

### (EXAMPLE 3)

1,3,4,6,8,9-hexahydroxyphenazine-2,7-dione, which is a partial reduced form of phenazine-1,2,3,4,6,7,8,9-octaone, was synthesized according to the procedure described in Chem. Commun., 2016, 52, 922-925. The details of the reaction conditions were determined based on the above-described literature. In addition, commercially available reagents were used for various reagents used in the synthesis.

Specifically, similarly to Example 2, first, 2,3,4,5-tetramethoxyaniline was dissolved in DMF, which was then heated and stirred together with an appropriate catalyst. Then, 1,2,3,6,7,8-hexamethoxyphenazine and (E)-1,2-bis (2,3,4,5-tetramethoxyphenyl) diazene were obtained by a separation operation using column chromatography.

Next, the separated (E)-1,2-bis(2,3,4,5-tetramethoxyphenyl) diazene was dissolved in a mixed solution of an acetic acid-hydrobromic acid, which was then heated and stirred at a reflux temperature. Thereafter, after cooling the solution to the room temperature, 1,3,4,6,8,9-hexahydroxyphenazine-2,7-dione was obtained by a filtration operation.

### (COMPARATIVE EXAMPLE 1)

A naphthazarin dimer represented by the following chemical formula was synthesized according to the procedure described in Yao, M., Taguchi, N., Ando, H. et al. "Improved gravimetric energy density and cycle life in organic lithium-ion battery with naphthazarin-based electrode materials", Commun Mater 1, 70 (2020). The details of the reaction conditions were determined based on the above-described literature. In addition, commercially available reagents were used for various reagents used in the synthesis.

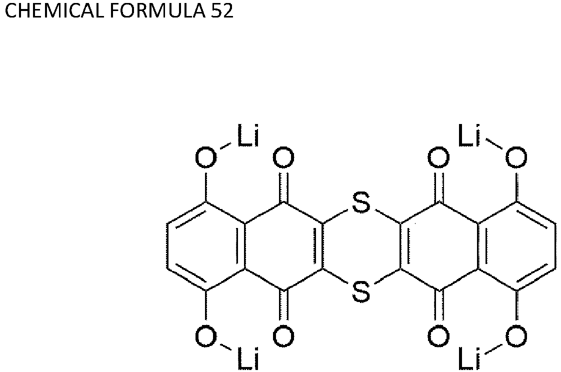

Specifically, first, 2,3-dichloronaphtazarin was prepared as a starting material. Next, the hydroxyl group of 2,3-dichloronaphtazarin was protected with an acetyl group by a reflux operation using acetic anhydride. Next, a dimerization reaction using rubeanic acid was performed at the condition of 50°C under presence of triethylamine (TEA) and N,N-dimethylformamide (DMF). Thereby, a chemical compound having a dibenzo[b,i]thianthrene skeleton was obtained. Then, hydrolysis was conducted at the condition of 70°Cunder presence of tetrahydrofuran. In addition, neutralization was conducted using lithium hydroxide. Thereby, the naphthazarin dimer was obtained.

### (TEST EXAMPLE 1)

An R2032 coin-type battery was manufactured using phenazine-1,4,6,9-tetraone synthesized in Example 1 as a positive electrode active material. The R2032 coin-type battery was manufactured according to the following procedure.

First, phenazine-1,4,6,9-tetraone as a positive electrode active material, acetylene black (available from Denka Co., Ltd.) as a conductive additive, and PTFE (available from Daikin Industries, Ltd.) as a binder were mixed in a ratio of 4:5:1 (mass ratio) (=positive electrode active material: conductive additive: binder) to manufacture a positive electrode active material sheet having a diameter of 10 mm and a thickness of 100 µm. A positive electrode was manufactured by pressing the positive electrode active material sheet described above to a stainless steel mesh (SUS304 available from Nilaco Co., Ltd.) having a diameter of 14 mm and a thickness of 100 µm.

Next, a circular member having a diameter of 13 mm was cut out from a 0.5 mm thick metal lithium foil (available from Honjo Metal Co., Ltd., purity 99.8% or higher). A negative electrode was manufactured by pressing the member cut out from the lithium foil onto a stainless steel plate (available from Hohsen Corp.) having a diameter of 15.5 mm and a thickness of 0.5 mm.

Further, as a separator, a glass filter (available from Advantech Co., Ltd.) having a diameter of 16 mm and a thickness of 0.4 mm was prepared. As an electrolyte solution, a non-aqueous electrolyte solution (available from Kishida Chemical Co., Ltd.) including LiPF₆ and a mixture of ethylene carbonate and diethyl carbonate was prepared. In a battery case conforming to the R2032 coin-type battery standard, the positive electrode, the separator, the negative electrode, and the electrolyte solution were arranged to manufacture a coin-type battery for a test.

The mass of phenazine-1,4,6,9-tetraone included in the coin-type battery for a test was 3 mg. In addition, the mass of the coin-type battery for a test was 3.1 g. For the manufactured coin-type battery for a test, the charging/discharging test was conducted within a potential range of 4.5-1.2 V (vs. Li⁺/Li) at a current density of 20 mA/g under atmosphere of 30°C.

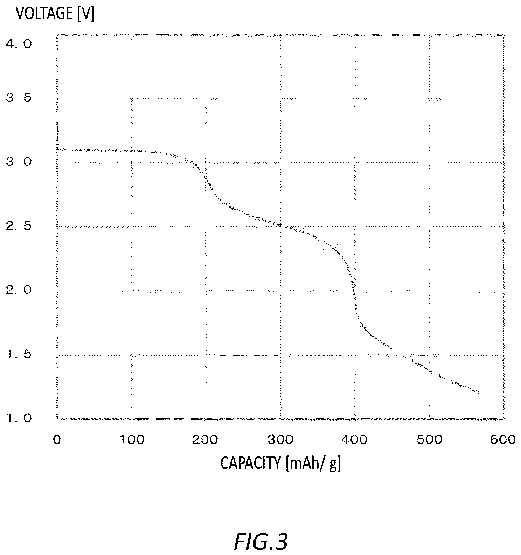

FIG. 3 represents an initial discharging curve. In addition, the initial discharging capacity was 580 [mAh/g-positive electrode active material]. The energy density of the coin-type battery for a test was 1.1 to 1.4 [Wh/g-coin-type battery].

### (TEST EXAMPLE 2)

An R2032 coin-type battery was manufactured using the reduced form of phenazine-2,3,4,7,8,9-hexaone synthesized in Example 2 as a positive electrode active material. A coin-type battery for a test was manufactured according to the procedure similar to Test Example 1, except that (i) as a positive electrode active material, the reduced form of phenazine-2,3,4,7,8,9-hexaone synthesized in Example 2 was used, and (ii) as an electrolyte solution, a non-aqueous electrolyte solution (available from Kishida Chemical Co., Ltd.) including lithium bis(trifluoromethanesulfonyl)imide (LiTFSI)) and sulfolane (SL) was used.

The mass of the reduced form of phenazine-2,3,4,7,8,9-hexaone included in the coin-type battery for a test was 1 mg. In addition, the mass of the coin-type battery for a test was 3.2 g. For the manufactured coin-type battery for a test, the charging/discharging test was conducted within a potential range of 4.5-1.2 V (vs. Li⁺/Li) at a current density of 20 mA/g under atmosphere of 30°C.

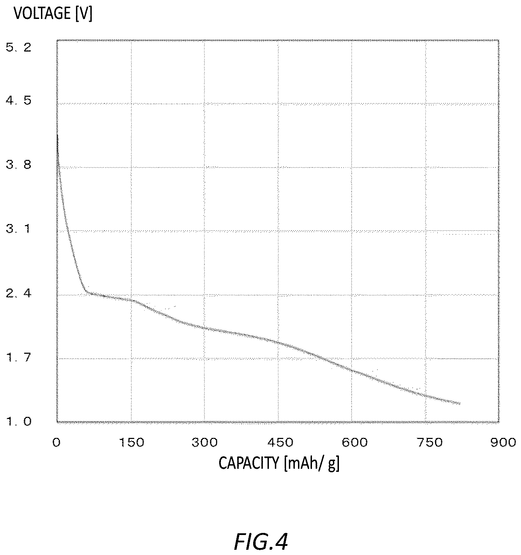

FIG. 4 represents an initial discharging curve. In addition, the initial discharging capacity was 820 [mAh/g-positive electrode active material]. The energy density of the coin-type battery for a test was 1.5 [Wh/g-coin-type battery]. Note that it is considered that the discharging capacity includes contributions from components other than the active material.

### (TEST EXAMPLE 3)

An R2032 coin-type battery was manufactured using the partial reduced form of phenazine-1,2,3,4,6,7,8,9-octaone synthesized in Example 3 as a positive electrode active material. A coin-type battery for a test was manufactured according to the procedure similar to Test Example 2, except that
the partial reduced form of phenazine-1,2,3,4,6,7,8,9-octaone synthesized in Example 3 was used as the positive electrode active material.

The mass of the partial reduced form of phenazine-1,2,3,4,6,7,8,9-octaone included in the coin-type battery for a test was 2 mg. In addition, the mass of the coin-type battery for a test was 3.2 g. For the manufactured coin-type battery for a test, the charging/discharging test was conducted within a potential range of 4.5-1.2 V (vs. Li⁺/Li) at a current density of 20 mA/g under atmosphere of 30°C.

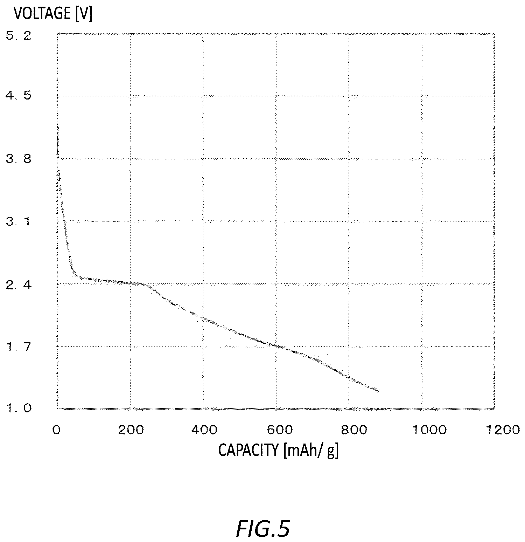

FIG. 5 represents an initial discharging curve. In addition, the initial discharging capacity was 880 [mAh/g-positive electrode active material]. The energy density of the coin-type battery for a test was 1.7 [Wh/g-coin-type battery]. Note that it is considered that the discharging capacity includes contributions from components other than the active material.

### (TEST EXAMPLE 4)

An R2032 coin-type battery was produced using the naphthazarin dimer synthesized in Comparative Example 1 as a positive electrode active material. The R2032 coin-type battery was manufactured according to the following procedure.

First, naphthazarin dimer as a positive electrode active material, acetylene black (available from Denka Co., Ltd.) and carbon nanotubes (available from JiangSu Cnano Technology Ltd.) as conductive additives, and PVDF (available from Kureha Co., Ltd.) as a binder were mixed to manufacture a slurry composition. The positive electrode active material, the acetylene black, the carbon nanotubes, and the binder were mixed in a ratio of 100:14:3:4 (mass ratio) (=positive electrode active material: acetylene black: carbon nanotubes: binder). In addition, the ratio of the positive electrode active material, acetylene black, carbon nanotubes, and binder in the slurry composition was 83 mass%.

Next, the slurry composition was applied to an aluminum foil (Hohsen Corp., thickness: 20 µm) subjected to a surface roughening treatment. After drying the slurry composition, it was punched out with 14 mmφ to manufacture a positive electrode. The thickness of the positive electrode was 134 µm. The application amount of naphthazarin dimer was 4.3 mg/cm².

Next, a circular member having a diameter of 13 mm was cut out from a 0.5 mm thick metal lithium foil (available from Honjo Metal Co., Ltd., purity 99.8% or higher). A negative electrode was manufactured by pressing the member cut out from the lithium foil onto a stainless steel plate (available from Hohsen Corp.) having a diameter of 15.5 mm and a thickness of 0.5 mm.

Further, as a separator, a glass filter paper (available from Advantech Co., Ltd.) having a diameter of 16 mm and a thickness of 0.4 mm was prepared. As the electrolyte solution, a non-aqueous electrolyte solution (available from Kishida Chemical Co., Ltd.) including LiTFSI and SL was prepared. In a battery case conforming to the R2032 coin-type battery standard, the positive electrode, the separator, the negative electrode, and the electrolyte solution were arranged to manufacture a coin-type battery for a test.

The mass of the naphthazarin dimer included in the coin-type battery for a test was 3 mg. In addition, the mass of the coin-type battery for a test was 3.2 g. For the manufactured coin-type battery for a test, the charging/discharging test was conducted within a potential range of 4.5-1.2 V (vs. Li⁺/Li) at a current density of 20 mA/g under atmosphere of 30°C.

The initial discharging capacity was 416 [mAh/g-positive electrode active material]. The energy density of the coin-type battery for a test was 1.1 [Wh/g-active material].

As shown in Test Examples 1 to 4, the secondary batteries using the phenazines synthesized in Examples 1 to 3 as the positive electrode active material each have a very high energy density, as compared with the secondary battery using the naphthazarine dimer synthesized in Comparative Example 1 as the positive electrode active material. In addition, it can be seen that the secondary battery using the various phenazines described above as the positive electrode active material are particularly suitable for use as a storage cell or a storage battery mounted on a flying body because the amount of electric charges that can be accumulated per unit mass is large.

In the case of the secondary battery using the naphthazarin dimer synthesized in Comparative Example 1 as a positive electrode active material, the theoretical capacity of the naphthazarin dimer is 462 [mhA/g], and the energy density of the active material single body obtained by multiplying 2.7 V of the average potential based on Li is 1.3 W/g as the maximum. In addition, the value actually observed is 1.1 Wh/g. For this reason, it is assumed that the upper limit of the energy density of the secondary battery is about 400 [Wh/kg-storage cell].

On the other hand, in the chemical compounds synthesized in Examples 1 to 3, there are few atoms not directly involved in oxidation-reduction in the molecule. In addition, the chemical compounds synthesized in Examples 1 to 3 do not include an atom having a relatively large atomic weight, such as sulfur. As a result, it is estimated that the theoretical capacity of the chemical compounds synthesized in Examples 1 to 3 was larger than that of the naphthazarin dimer. Since the energy density of the active material is calculated as the product of the theoretical capacity and the average discharging potential, it is estimated that the energy density of the chemical compounds synthesized in Examples 1 to 3 was also higher than that of the naphthazarin dimer.

As described above, the chemical compound represented by any of the general formula (1) to the general formula (7) has few atoms not directly involved in oxidation-reduction in the molecule, similar to the chemical compounds synthesized in Examples 1 to 3. In addition, the chemical compound represented by any of the general formula (1) to the general formula (7) does not include an atom having a relatively large atomic weight, such as sulfur, similarly to the chemical compounds synthesized in Examples 1 to 3. For this reason, by using the chemical compound represented by any of the general formula (1) to the general formula (7) or a salt thereof as an active material for a secondary battery (particularly, a positive electrode active material), a storage cell having an energy density of 500 [Wh/kg-storage cell] or higher can be manufactured.

Similarly, the polymer including the repeating unit represented by any of the general formula (8) to the general formula (11) described above has few atoms not directly involved in oxidation-reduction in the molecule, similar to the chemical compounds synthesized in Examples 1 to 3. In addition, the polymer including the repeating unit represented by any of the general formula (8) to the general formula (11) does not include an atom having a relatively large atomic weight, such as sulfur, similarly to the chemical compounds synthesized in Examples 1 to 3. For this reason, by using the polymer including the repeating unit represented by any of the general formula (8) to the general formula (11) or a salt thereof as an active material for a secondary battery (particularly, a positive electrode active material), a storage cell having an energy density of 500 [Wh/kg-storage cell] or higher can be manufactured.

While the embodiments of the present invention have been described, the technical scope of the present invention is not limited to the above-described embodiments. It is apparent to persons skilled in the art that various alterations or improvements can be made to the above-described embodiments. It is also apparent from the scope of the claims that the embodiments added with such alterations or improvements can be included in the technical scope of the present invention.

The operations, procedures, steps, stages etc. of every processing performed by an apparatus, system, program, and method shown in the claims, specification, or diagrams can be performed in any order as long as the order is not indicated by "prior to," "before," or the like and as long as the output from a previous processing is not used in a later processing. Even if the operation flow is described with phrases such as "first" or "next" in the claims, specification, or diagrams, they do not necessarily mean that the flow must be performed in this order.

### EXPLANATION OF REFERENCES

100: flying body, 110: storage battery, 112: storage cell, 120: electrical power control circuit, 130: electric motor, 140: propeller, 150: sensor, 160: control apparatus, 212: positive electrode case, 214: negative electrode case, 216: sealant, 218: metal spring, 220: positive electrode, 222: positive electrode current collector, 224: positive electrode active material layer, 230: separator, 240: negative electrode, 242: negative electrode current collector, 244: negative electrode active material layer, 250: electrolyte solution.

## Claims

1. An active material for a secondary battery that is used as an active material of a secondary battery, the active material comprising:
a chemical compound represented by a general formula (1), general formula (2), general formula (3), general formula (4), general formula (4), general formula (5), general formula (6) or general formula (7) below, or a salt thereof, 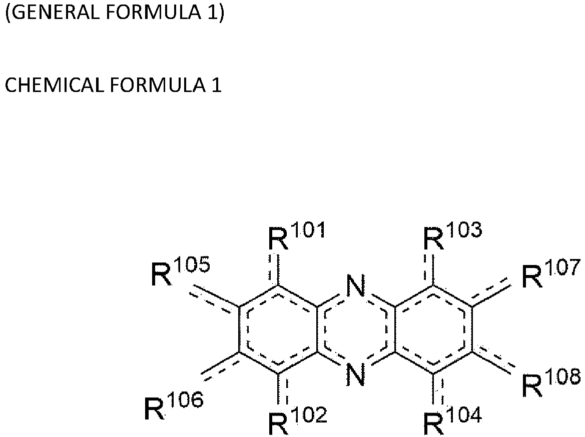
(in the general formula (1), R¹⁰¹ to R¹⁰⁸ each independently represent an oxygen atom, a group represented by -OM, a hydrogen atom or an organic group.
M represents a hydrogen atom or a monovalent or bivalent metal atom.
Note that, when M is a bivalent metal atom, two adjacent R¹⁰¹ to R¹⁰⁸ are coupled to each other to form a ring represented by -OMO-.
A double line represented by a solid line and a dashed line represents a single bond or a double bond.
At least four of R¹⁰¹ to R¹⁰⁸ are oxygen atoms, or groups represented by -OM.) 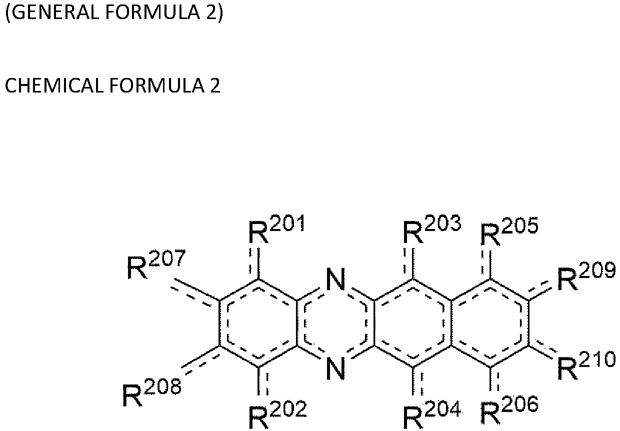
(in the general formula (2), R²⁰¹ to R²¹⁰ each independently represent an oxygen atom, a group represented by -OM, a hydrogen atom or an organic group.
M represents a hydrogen atom or a monovalent or bivalent metal atom.
Note that, when M is a bivalent metal atom, two adjacent R²⁰¹ to R²¹⁰ are coupled to each other to form a ring represented by -OMO-.
A double line represented by a solid line and a dashed line represents a single bond or a double bond.
At least four of R²⁰¹ to R²¹⁰ are oxygen atoms, or groups represented by -OM.) 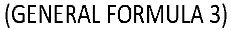 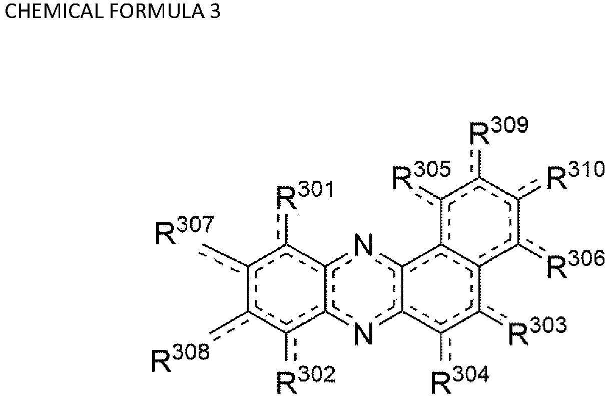
(in the general formula (3), R³⁰¹ to R³¹⁰ each independently represent an oxygen atom, a group represented by -OM, a hydrogen atom or an organic group.
M represents a hydrogen atom or a monovalent or bivalent metal atom.
Note that, when M is a bivalent metal atom, two adjacent R³⁰¹ to R³¹⁰ are coupled to each other to form a ring represented by -OMO-.
A double line represented by a solid line and a dashed line represents a single bond or a double bond.
At least four of R³⁰¹ to R³¹⁰ are oxygen atoms, or groups represented by -OM.) 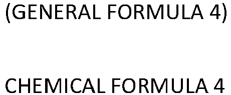 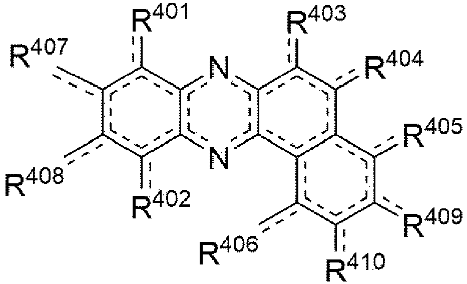
(in the general formula (4), R⁴⁰¹ to R⁴¹⁰ each independently represent an oxygen atom, a group represented by -OM, a hydrogen atom or an organic group.
M represents a hydrogen atom or a monovalent or bivalent metal atom.
Note that, when M is a bivalent metal atom, two adjacent R⁴⁰¹ to R⁴¹⁰ are coupled to each other to form a ring represented by -OMO-.
A double line represented by a solid line and a dashed line represents a single bond or a double bond.
At least four of R⁴⁰¹ to R⁴¹⁰ are oxygen atoms, or groups represented by -OM.) 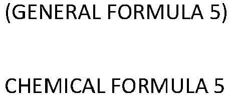 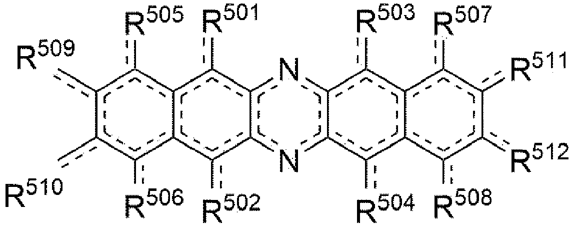 (in the general formula (5), R⁵⁰¹ to R⁵¹² each independently represent an oxygen atom, a group represented by -OM, a hydrogen atom or an organic group.
M represents a hydrogen atom or a monovalent or bivalent metal atom.
Note that, when M is a bivalent metal atom, two adjacent R⁵⁰¹ to R⁵¹² are coupled to each other to form a ring represented by -OMO-.
A double line represented by a solid line and a dashed line represents a single bond or a double bond.
At least four of R⁵⁰¹ to R⁵¹² are oxygen atoms, or groups represented by -OM.) 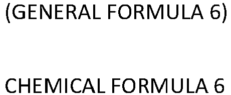 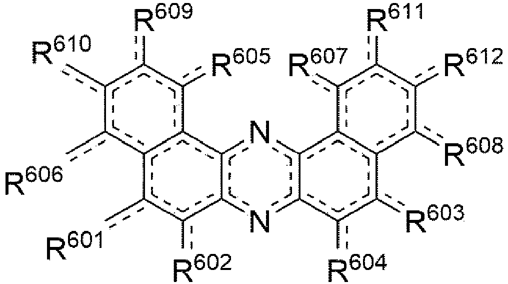
(in the general formula (6), R⁶⁰¹ to R⁶¹² each independently represent an oxygen atom, a group represented by -OM, a hydrogen atom or an organic group.
M represents a hydrogen atom or a monovalent or bivalent metal atom.
Note that, when M is a bivalent metal atom, two adjacent R⁶⁰¹ to R⁶¹² are coupled to each other to form a ring represented by -OMO-.
A double line represented by a solid line and a dashed line represents a single bond or a double bond.
At least four of R⁶⁰¹ to R⁶¹² are oxygen atoms, or groups represented by -OM.) 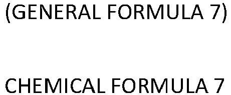 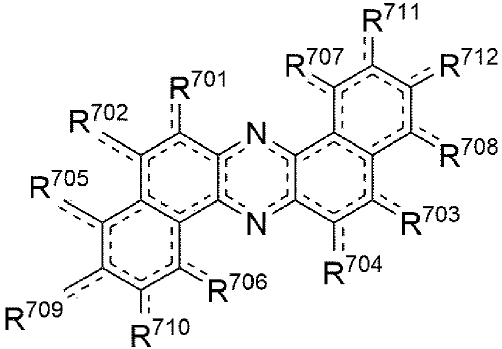
(in the general formula (7), R⁷⁰¹ to R⁷¹² each independently represent an oxygen atom, a group represented by -OM, a hydrogen atom or an organic group.
M represents a hydrogen atom or a monovalent or bivalent metal atom.
Note that, when M is a bivalent metal atom, two adjacent R⁷⁰¹ to R⁷¹² are coupled to each other to form a ring represented by -OMO-.
A double line represented by a solid line and a dashed line represents a single bond or a double bond.
At least four of R⁷⁰¹ to R⁷¹² are oxygen atoms, or groups represented by -OM.)

2. The active material for the secondary battery according to Claim 1, wherein the chemical compound has symmetry.

3. The active material for the secondary battery according to Claim 1, wherein the chemical compound is 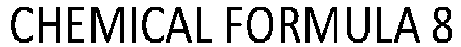 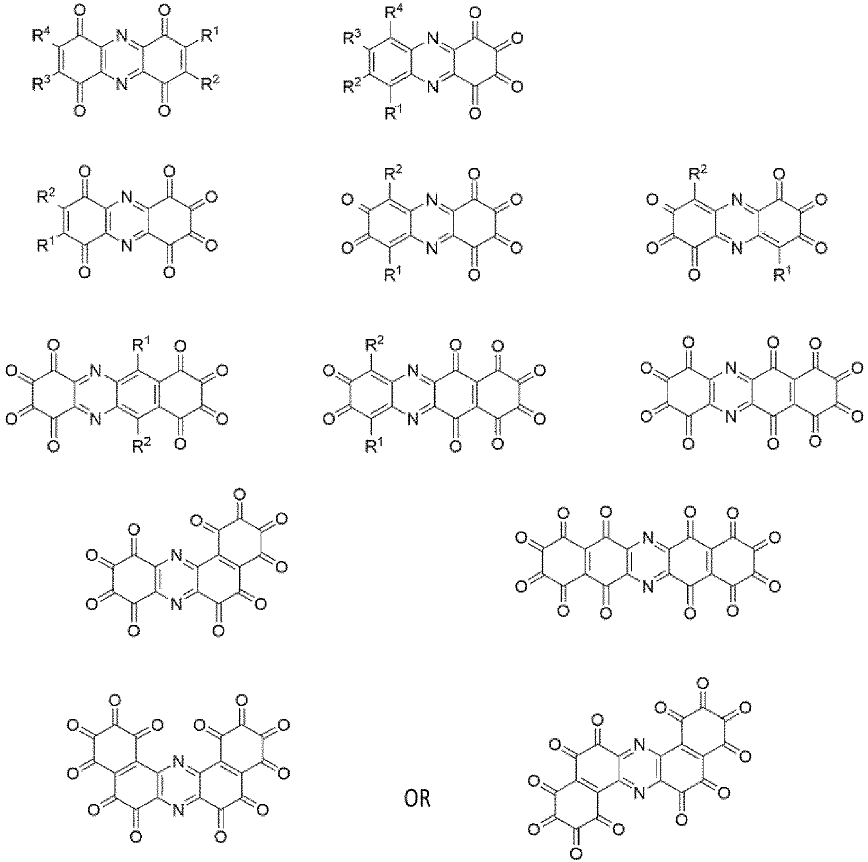
or a fully reduced form or partial reduced form thereof, and
in the chemical formula, R¹ and R² each independently represent a hydrogen atom or an organic group.

4. An active material for a secondary battery that is used as an active material of a secondary battery, the active material comprising:
a polymer including a repeating unit represented by a general formula (8), general formula (9), general formula (10) or general formula (11) below, or a salt thereof, 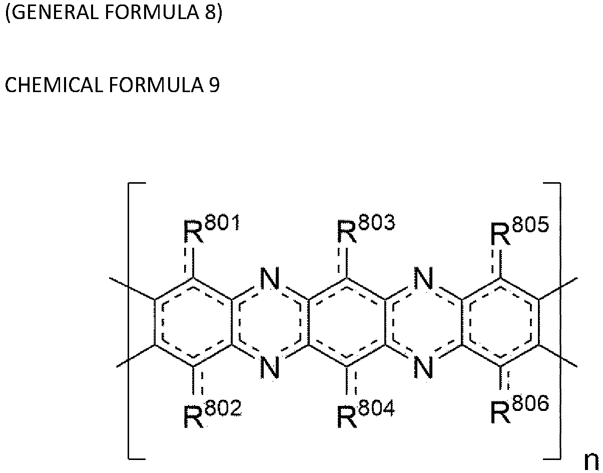
(in the general formula (8), R⁸⁰¹ to R⁸⁰⁶ each independently represent an oxygen atom, a group represented by -OM, a hydrogen atom or an organic group.
M represents a hydrogen atom or a monovalent or bivalent metal atom.
Note that, when M is a bivalent metal atom, two adjacent R⁸⁰¹ to R⁸⁰⁶ are coupled to each other to form a ring represented by -OMO-.
A double line represented by a solid line and a dashed line represents a single bond or a double bond.
At least four of R⁸⁰¹ to R⁸⁰⁶ are oxygen atoms, or groups represented by -OM.
n is an integer of 2 or larger representing a polymerization degree.) 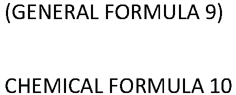 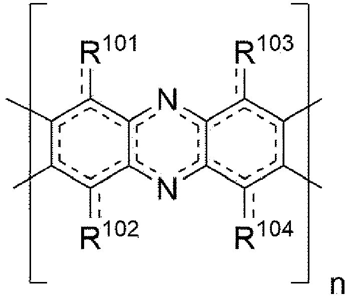
(in the general formula (9), R¹⁰¹ to R¹⁰⁴ each independently represent an oxygen atom or a group represented by -OM.
M represents a hydrogen atom or a monovalent or bivalent metal atom.
Note that, when M is a bivalent metal atom, two adjacent R¹⁰¹ to R¹⁰⁴ are coupled to each other to form a ring represented by -OMO-.
A double line represented by a solid line and a dashed line represents a single bond or a double bond.
n is an integer of 2 or larger representing a polymerization degree.) 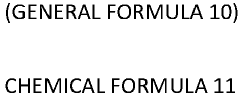 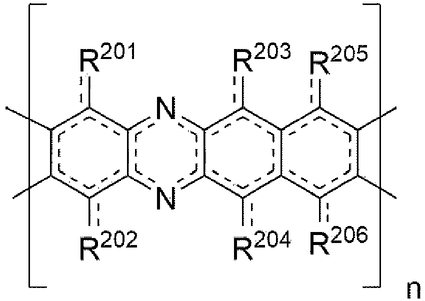
(in the general formula (10), R²⁰¹ to R²⁰⁶ each independently represent an oxygen atom, a group represented by -OM, a hydrogen atom or an organic group.
M represents a hydrogen atom or a monovalent or bivalent metal atom.
Note that, when M is a bivalent metal atom, two adjacent R²⁰¹ to R²⁰⁶ are coupled to each other to form a ring represented by -OMO-.
A double line represented by a solid line and a dashed line represents a single bond or a double bond.
At least four of R²⁰¹ to R²⁰⁶ are oxygen atoms, or groups represented by -OM.
n is an integer of 2 or larger representing a polymerization degree.) 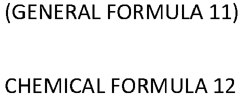 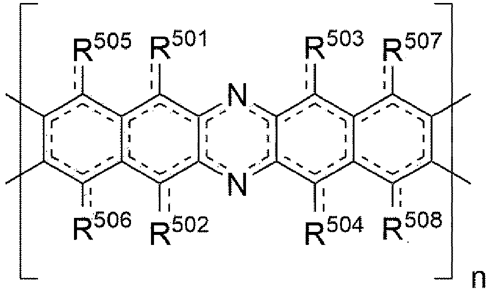
(in the general formula (11), R⁵⁰¹ to R⁵⁰⁸ each independently represent an oxygen atom, a group represented by -OM, a hydrogen atom or an organic group.
M represents a hydrogen atom or a monovalent or bivalent metal atom.
Note that, when M is a bivalent metal atom, two adjacent R⁵⁰¹ to R⁵⁰⁸ are coupled to each other to form a ring represented by -OMO-.
A double line represented by a solid line and a dashed line represents a single bond or a double bond.
At least four of R⁵⁰¹ to R⁵⁰⁸ are oxygen atoms, or groups represented by -OM.
n is an integer of 2 or larger representing a polymerization degree.)

5. The active material for the secondary battery according to Claim 4, wherein
the active material for a secondary battery comprises a polymer including a repeating unit represented by a following formula (8-1), or a salt thereof, 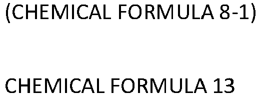 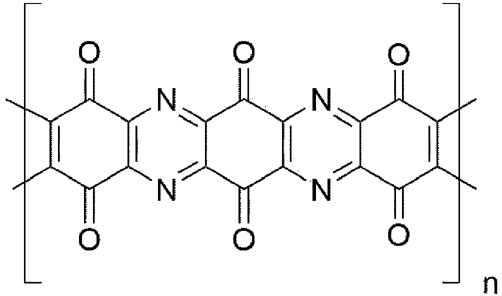
(in the chemical formula (8-1), n is an integer of 2 or larger representing a polymerization degree.)

6. The active material for the secondary battery according to any one of Claims 1 to 5, wherein M is H, Li, Na, K, Mg or Ca.

7. An electrode for a secondary battery comprising:
the active material for the secondary battery according to any one of Claims 1 to 6.

8. A secondary battery comprising:
a positive electrode active material layer including a positive electrode active material;
a negative electrode active material layer including a negative electrode active material; and
an electrolyte, wherein
the positive electrode active material or the negative electrode active material includes the active material for the secondary battery according to any one of Claims 1 to 6.

9. The secondary battery according to Claim 8, wherein
the positive electrode active material includes the active material for the secondary battery according to any one of Claims 1 to 6.

10. The secondary battery according to Claim 8 or 9, wherein
the secondary battery is a non-aqueous secondary battery.

11. A flying body comprising:
the secondary battery according to any one of Claims 8 to 10; and
a propulsive force generating apparatus configured to generate propulsive force by using electric energy accumulated in the secondary battery.
